(19) Europäisches Patentamt
European Patent Office
Office européen des brevets

(11) EP 4 685 063 A1

# (12) EUROPEAN PATENT APPLICATION
published in accordance with Art. 153(4) EPC

(43) Date of publication:
**28.01.2026 Bulletin 2026/05**

(21) Application number: **24774593.8**

(22) Date of filing: **27.02.2024**

(51) International Patent Classification (IPC):
***B65D 1/00*** *(2006.01)* ***B29B 17/04*** *(2006.01)*
***B32B 1/00*** *(2024.01)* ***B32B 27/18*** *(2006.01)*
***B32B 27/34*** *(2006.01)* ***B32B 27/36*** *(2006.01)*
***B65D 65/40*** *(2006.01)* ***C07C 237/30*** *(2006.01)*
***C07F 9/6574*** *(2006.01)* ***C08J 11/04*** *(2006.01)*

(52) Cooperative Patent Classification (CPC):
**B29B 17/04; B32B 1/00; B32B 27/18; B32B 27/34; B32B 27/36; B65D 1/00; B65D 65/40; C07C 237/30; C07F 9/6574; C08J 11/04**

(86) International application number:
**PCT/JP2024/007032**

(87) International publication number:
**WO 2024/195448 (26.09.2024 Gazette 2024/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.03.2023 JP 2023045784**

(71) Applicant: **MITSUBISHI GAS CHEMICAL COMPANY, INC.**
**Chiyoda-ku**
**Tokyo 100-8324 (JP)**

(72) Inventors:
- **YAMADA, Takumi**
**Hiratsuka-shi, Kanagawa 254-0016 (JP)**
- **KOBAYASHI, Masayuki**
**Hiratsuka-shi, Kanagawa 254-0016 (JP)**
- **OTSUKA, Kosuke**
**Tokyo 100-8324 (JP)**

(74) Representative: **Vossius & Partner Patentanwälte Rechtsanwälte mbB**
**Siebertstrasse 3**
**81675 München (DE)**

## (54) MULTILAYERED CONTAINER AND METHOD FOR PRODUCING RECYCLED POLYESTER

(57) A multilayer container includes: a polyester layer containing a polyester resin (X), a phenolic antioxidant (A), and a phosphorus-based antioxidant (B); or a polyester layer containing a polyester resin (X), a phenolic antioxidant (A), a phosphorus-based antioxidant (B), and an aldehyde catcher (C); and a polyamide layer containing a polyamide resin (Y). A total content of the phenolic antioxidant (A), the phosphorus-based antioxidant (B), and the aldehyde catcher (C) in the polyester layer is from 0.05 to 0.22 mass%, and the phosphorus-based antioxidant (B) is a compound having a pentaerythritol skeleton and an aromatic ring.



Processed by Luminess, 75001 PARIS (FR)

## Description

### Technical Field

The present invention relates to a multilayer container and a method for producing a recycled polyester.

### Background Art

Aromatic polyester resins obtained by using an aromatic dicarboxylic acid compound and an aliphatic diol compound as monomers have an advantage of being excellent in transparency, mechanical performance, melt stability, solvent resistance, fragrance preservation, gas barrier properties, recyclability, and the like. Therefore, aromatic polyester resins such as polyethylene terephthalate (PET) are widely used in various packaging materials such as films, sheets, and hollow containers. Polyester resins have high gas barrier properties, but are not always sufficient for applications requiring further gas barrier properties against oxygen, carbon dioxide, and the like. For this reason, as ways for improving the gas barrier properties of the polyester resin, there are performed: vapor-deposition of aluminum oxide or silicon oxide on a molded article or a packaging container made of a polyester resin; or an application, lamination, or melt-mixing of a resin having high gas barrier performance on a molded article or a packaging container made of a polyester resin.

Examples of the gas barrier resin include: polyamide resins such as nylon 6 and nylon 66; and ethylene-vinyl alcohol copolymers. Among the polyamide resins, a xylylene group-containing polyamide resin, which is obtained by polymerizing a diamine component containing xylylenediamine as a main component and a dicarboxylic acid component containing an aliphatic dicarboxylic acid as a main component, is particularly excellent in gas barrier properties. The xylylene group-containing polyamide resin has high gas barrier properties, and also whose glass transition temperature, melting point, and crystallinity are similar to those of polyethylene terephthalate that is a widely used polyester resin, and hence the xylylene group-containing polyamide resin is easily laminated on and melt-mixed with the polyester resin. For this reason, the xylylene group-containing polyamide resin is very suitable as a material for improving the gas barrier properties of the polyester resin.

However, yellowing due to thermal history is prone to proceed in a multilayer polyester container with a polyamide layer, as compared with polyester alone. Therefore, yellowing occurs particularly in a recycling process to recover containers and reuse the resin. This is a factor that reduces the commodity value of the packaging container, and thus, studies have been made to suppress yellowing.

For example, Patent Document 1 discloses a multilayer container including: a polyester resin composition layer containing a polyester resin and a specific amount of an antioxidant; and a polyamide resin composition layer containing a specific polyamide resin and a specific amount of a cobalt salt, for the purpose of suppressing yellowing of recycled polyester during recycling.

### Citation List

### Patent Document

Patent Document 1: JP 2018-043773 A

### Summary of Invention

### Technical Problem

As described above, yellowing is prone to proceed in the polyester resin container containing a polyamide, and recycled polyester obtained by recycling the container also is yellowish in color. As a method for suppressing the yellowing, for example, addition of an antioxidant to the container or the like has been performed as described in Patent Document 1, but colorlessness of the resultant recycled polyester has not yet been sufficient. Furthermore, the recycled polyester undergoes a recycling step, and thus haze tends to occur and improvement in transparency has also been required.

Therefore, an object of the present invention is to provide a multilayer container, recycling of which can obtain a recycled polyester that can be suppressed from yellowing and also is excellent in transparency.

### Solution to Problem

As a result of intensive studies in view of the above-mentioned problems, the present inventors have found that a multilayer container including a polyamide layer and further including a polyester layer containing a phenolic antioxidant and a specific phosphorus-based antioxidant can solve the above-mentioned problems, and have completed the present

invention.

The present invention provides the following [1] to [18].

[1] A multilayer container including: a polyester layer containing a polyester resin (X), a phenolic antioxidant (A), and a phosphorus-based antioxidant (B); or a polyester layer containing a polyester resin (X), a phenolic antioxidant (A), a phosphorus-based antioxidant (B), and an aldehyde catcher (C); and a polyamide layer containing a polyamide resin (Y), wherein a total content of the phenolic antioxidant (A), the phosphorus-based antioxidant (B) and the aldehyde catcher (C) in the polyester layer is from 0.05 to 0.22 mass%, and the phosphorus-based antioxidant (B) is a compound having a pentaerythritol skeleton and an aromatic ring.

[2] The multilayer container according to [1], wherein a total content of the phenolic antioxidant (A) and the phosphorus-based antioxidant (B) in the polyester layer is from 0.05 to 0.22 mass%.

[3] The multilayer container according to [1] or [2], wherein a mass ratio [(A)/(B)] of a content of the phenolic antioxidant (A) to a content of the phosphorus-based antioxidant (B) is from 2/8 to 5/5.

[4] The multilayer container according to any one of [1] to [3], wherein the polyester layer contains an aldehyde catcher (C).

[5] The multilayer container according to any one of [1] to [4], wherein the aldehyde catcher (C) is anthranilamide.

[6] The multilayer container according to any one of [1] to [5], wherein a content of the aldehyde catcher (C) in the polyester layer is from 0.005 to 0.120 mass%.

[7] The multilayer container according to any one of [1] to [6], wherein the polyamide resin (Y) contains a structural unit derived from a diamine and a structural unit derived from a dicarboxylic acid, 80 mol% or more of the structural unit derived from a diamine being a structural unit derived from xylylenediamine, and 80 mol% or more of the structural unit derived from a dicarboxylic acid being a structural unit derived from adipic acid.

[8] The multilayer container according to any one of [1] to [7], wherein a content of the polyamide resin (Y) is from 0.05 to 10.0 mass% relative to a total amount of all polyamide layers and all polyester layers.

[9] The multilayer container according to any one of [1] to [8], wherein a content of the polyamide layer is from 0.05 to 10.0 mass% relative to a total amount of all polyamide layers and all polyester layers.

[10] The multilayer container according to any one of [1] to [9], wherein the polyester resin (X) contains a structural unit derived from a dicarboxylic acid and a structural unit derived from a diol, 80 mol% or more of the structural unit derived from a dicarboxylic acid being a structural unit derived from terephthalic acid, and 80 mol% or more of the structural unit derived from a diol being a structural unit derived from ethylene glycol.

[11] The multilayer container according to any one of [1] to [10], wherein the multilayer container is a hollow container.

[12] The multilayer container according to any one of [1] to [11], wherein the multilayer container has a three- to five-layer structure, and the outermost layer and the innermost layer are polyester layers.

[13] A multilayer container including: a polyester layer containing a polyester resin (X), a phenolic antioxidant (A), a phosphorus-based antioxidant (B), and an aldehyde catcher (C); and a polyamide layer containing a polyamide resin (Y), wherein the phosphorus-based antioxidant (B) is a compound having a pentaerythritol skeleton and an aromatic ring, and the aldehyde catcher (C) is anthranilamide, a total content of the phenolic antioxidant (A), the phosphorus-based antioxidant (B), and the aldehyde catcher (C) in the polyester layer is from 0.05 to 0.22 mass%, a content of the aldehyde catcher (C) in the polyester layer is from 0.005 to 0.120 mass%, and a mass ratio [(A)/(B)] of a content of the phenolic antioxidant (A) to a content of the phosphorus-based antioxidant (B) is from 2/8 to 5/5.

[14] A method for producing a recycled polyester, the method including recovering polyester from the multilayer container according to any one of [1] to [13].

[15] The method for producing a recycled polyester according to [14], wherein the polyester is subjected to one or more steps selected from crystallizing and solid-phase polymerizing after the recovering.

[16] The method for producing a recycled polyester according to [14] or [15], the method including removing the whole or a part of the polyamide layer from the multilayer container to recover the polyester.

[17] The method for producing a recycled polyester according to [16], wherein the removing of the polyamide layer is conducted by, after milling of the multilayer container, air elutriation.

[18] The method for producing a recycled polyester according to any one of [14] to [17], wherein the method includes washing the multilayer container or a milled product thereof with an alkaline aqueous solution to recover the polyester.

Advantageous Effects of Invention

According to the present invention, it is possible to provide a multilayer container, recycling of which can obtain a recycled polyester that can be suppressed from yellowing and also is excellent in transparency.

Description of Embodiments

## Multilayer container

The multilayer container of the present invention is a multilayer container including: a polyester layer containing a polyester resin (X), a phenolic antioxidant (A), and a phosphorus-based antioxidant (B); or a polyester layer containing a polyester resin (X), a phenolic antioxidant (A), a phosphorus-based antioxidant (B), and an aldehyde catcher (C); and a polyamide layer containing a polyamide resin (Y), in which the total content of the phenolic antioxidant (A), the phosphorus-based antioxidant (B), and the aldehyde catcher (C) in the polyester layer is from 0.05 to 0.22 mass%, and the phosphorus-based antioxidant (B) is a compound having a pentaerythritol skeleton and an aromatic ring.

More specifically, the multilayer container of the present invention includes a multilayer container including: a polyester layer containing a polyester resin (X), a phenolic antioxidant (A), and a phosphorus-based antioxidant (B) but not containing an aldehyde catcher (C); and a polyamide layer containing a polyamide resin (Y), in which a total content of the phenolic antioxidant (A) and the phosphorus-based antioxidant (B) in the polyester layer is from 0.05 to 0.22 mass%, and the phosphorus-based antioxidant (B) is a compound having a pentaerythritol skeleton and an aromatic ring, furthermore, the multilayer container of the present invention includes a multilayer container including: a polyester layer containing a polyester resin (X), a phenolic antioxidant (A), a phosphorus-based antioxidant (B), and an aldehyde catcher (C); and a polyamide layer containing a polyamide resin (Y), in which a total content of the phenolic antioxidant (A), the phosphorus-based antioxidant (B) and the aldehyde catcher (C) in the polyester layer is from 0.05 to 0.22 mass%, and the phosphorus-based antioxidant (B) is a compound having a pentaerythritol skeleton and an aromatic ring.

Though the reason why a recycled polyester obtained by recycling the multilayer container of the present invention can be suppressed from yellowing and also is excellent in transparency, it may be assumed to be as follows. When an antioxidant is blended in a multilayer container in order to suppress yellowing during recycling, the antioxidant in the container is hydrolyzed or flows out during use of the container or during washing at recycling, and its effect is difficult to be fully achieved. As for the multilayer container of the present invention, it is believed that the influence of hydrolysis and the like can be suppressed by concomitant use of the phenolic antioxidant and the phosphorus-based antioxidant having a pentaerythritol skeleton and an aromatic ring, and even from the container including a polyamide layer, a recycled polyester excellent in colorlessness and transparency can be obtained.

### Polyester Layer

The polyester layer contains a polyester resin (X), a phenolic antioxidant (A), and a phosphorus-based antioxidant (B).

#### Polyester resin (X)

The polyester resin (X) contained in the polyester layer is preferably a polycondensation polymer of a dicarboxylic acid and a diol, and preferably includes a structural unit derived from a dicarboxylic acid (a dicarboxylic acid unit) and a structural unit derived from a diol (a diol unit).

Examples of dicarboxylic acid unit include a structural unit derived from an aromatic dicarboxylic acid, a structural unit derived from an alicyclic dicarboxylic acid, and a structural unit derived from an aliphatic dicarboxylic acid, and a structural unit derived from an aromatic dicarboxylic acid is preferable.

Examples of the aromatic dicarboxylic acid include terephthalic acid, isophthalic acid, orthophthalic acid, biphenyldicarboxylic acid, diphenyl ether dicarboxylic acid, diphenylsulfonedicarboxylic acid, diphenylketonedicarboxylic acid, 2,6-naphthalenedicarboxylic acid, 1,4-naphthalenedicarboxylic acid, and 2,7-naphthalenedicarboxylic acid, and from the viewpoints of cost and ease of production, terephthalic acid, isophthalic acid, orthophthalic acid, naphthalenedicarboxylic acid, and 4,4'-biphenyldicarboxylic acid are preferable, terephthalic acid, isophthalic acid, and naphthalenedicarboxylic acid are more preferable, from the viewpoints of moldability, terephthalic acid and isophthalic acid are still more preferable, and terephthalic acid is yet more preferable.

In recycling of the multilayer container of the present invention, the multilayer container may be melt-kneaded with a known typical single layer container made of a polyester resin. When a unit derived from terephthalic acid is contained as the dicarboxylic acid unit, the miscibility between the multilayer container of the present invention and the known single-layer container is improved, and the recyclability is improved.

As the aromatic dicarboxylic acid, sulfophthalic acid or a sulfophthalic acid metal salt may be used. The sulfophthalic acid metal salt is a metal salt of sulfophthalic acid, and examples of the metal atom include an alkali metal and an alkaline earth metal.

Examples of the alicyclic dicarboxylic acid include cyclohexanedicarboxylic acid, norbornene dicarboxylic acid, and tricyclodecane dicarboxylic acid.

Examples of the aliphatic dicarboxylic acid include malonic acid, succinic acid, adipic acid, azelaic acid, and sebacic acid.

Examples of the diol unit include a structural unit derived from an aliphatic diol, a structural unit derived from an alicyclic diol, and a structural unit derived from an aromatic diol, and a structural unit derived from an aliphatic diol is preferable.

Examples of the aliphatic diol include ethylene glycol, 2-butene-1,4-diol, trimethylene glycol, tetramethylene glycol, hexamethylene glycol, neopentyl glycol, methylpentanediol, and diethylene glycol. Among them, ethylene glycol is preferable.

Examples of the alicyclic diol include cyclohexanedimethanol, isosorbide, spiroglycol, 2,2,4,4-tetramethyl-1,3-cyclobutanediol, norbornene dimethanol, and tricyclodecane dimethanol.

Examples of the aromatic diol include a bisphenol compound and a hydroquinone compound.

The polyester resin (X) may have a structural unit derived from a hydroxycarboxylic acid.

Examples of the hydroxycarboxylic acid include aliphatic hydroxycarboxylic acids, alicyclic hydroxycarboxylic acids, and aromatic hydroxycarboxylic acids.

Examples of the aliphatic hydroxycarboxylic acid include 10-hydroxyoctadecanoic acid, lactic acid, hydroxyacrylic acid, 2-hydroxy-2-methylpropionic acid, and hydroxybutyl acid.

Examples of the alicyclic hydroxycarboxylic acid include hydroxymethyl cyclohexane carboxylic acid, hydroxymethyl norbornene carboxylic acid, and hydroxymethyl tricyclodecane carboxylic acid.

Examples of the aromatic hydroxycarboxylic acid include hydroxybenzoic acid, hydroxytoluic acid, hydroxynaphthoic acid, 3-(hydroxyphenyl)propionic acid, hydroxyphenylacetic acid, and 3-hydroxy-3-phenylpropionic acid.

The polyester resin (X) may have a structural unit derived from a monofunctional compound and a structural unit derived from a polyfunctional compound.

Examples of the monofunctional compound include monocarboxylic acids and monoalcohols, and specific examples thereof include aromatic monocarboxylic acids, aliphatic monocarboxylic acids, aromatic monoalcohols, aliphatic monoalcohols, and alicyclic monoalcohols.

Examples of the polyfunctional compound include aromatic polycarboxylic acids, alicyclic polycarboxylic acids, aliphatic polyhydric alcohols, alicyclic polyhydric alcohols, and ester forms thereof.

The polyester resin (X) preferably contains structural units derived from a dicarboxylic acid in which structural units derived from terephthalic acid are contained, and structural units derived from a diol in which structural units derived from ethylene glycol are contained, and more preferably 80 mol% or more of the structural units derived from a dicarboxylic acid are structural units derived from terephthalic acid, and 80 mol% or more of the structural units derived from a diol are structural units derived from ethylene glycol; still more preferably 90 mol% or more of the structural units derived from a dicarboxylic acid are structural units derived from terephthalic acid, and 90 mol% or more of the structural units derived from a diol are structural units derived from ethylene glycol; yet still more preferably 98 mol% or more of the structural units derived from a dicarboxylic acid are structural units derived from terephthalic acid, and substantially 100 mol% or more of the structural units derived from a diol are structural units derived from ethylene glycol.

Specific examples of the polyester resin (X) include polyethylene terephthalate (PET).

The polyethylene terephthalate (PET) may contain a structural unit derived from an aromatic dicarboxylic acid other than terephthalic acid. The aromatic dicarboxylic acid other than terephthalic acid is preferably one or more selected from the group consisting of isophthalic acid, orthophthalic acid, naphthalenedicarboxylic acid, and 4,4'-biphenyldicarboxylic acid. These compounds are at low cost, and a copolymerized polyester resin containing these compounds is easily produced.

Among them, isophthalic acid and naphthalenedicarboxylic acid are preferable, and isophthalic acid is more preferable. Polyethylene terephthalate containing a structural unit derived from isophthalic acid is excellent in moldability, and is also excellent in terms of preventing whitening of a molded article due to a slow crystallization rate. In addition, polyethylene terephthalate containing a naphthalenedicarboxylic acid-derived structural unit increases the glass transition point of the resin and thereby improving thermal resistance, and also absorbs ultraviolet rays, so that it is suitably used for production of a multilayer container required to have resistance to ultraviolet rays. Note that, as the naphthalenedicarboxylic acid, a 2,6-naphthalenedicarboxylic acid component is preferable because it is easy to produce and is highly economical.

When polyethylene terephthalate contains a structural unit derived from an aromatic dicarboxylic acid other than terephthalic acid, the proportion of the constitution derived from an aromatic dicarboxylic acid other than terephthalic acid is preferably from 1 to 20 mol%, more preferably from 1 to 10 mol%, and still more preferably from 1 to 5 mol% of the dicarboxylic acid unit.

Note that the polyester resin (X) may be used singly, or two or more types thereof may be used in combination.

The polyester resin (X) can be produced by a known method such as a direct esterification process or a transesterification process.

The intrinsic viscosity of the polyester resin (X) is preferably from 0.5 to 2.0 dL/g, and more preferably from 0.6 to 1.5 dL/g. When the intrinsic viscosity is 0.5 dL/g or more, the container is excellent in mechanical properties.

Note that the intrinsic viscosity is measured by a manner in which the polyester resin is dissolved in a mixed

solvent of phenol/1,1,2,2-tetrachloroethane (= 6/4 mass ratio) to prepare 0.2, 0.4, and 0.6 g/dL solutions, and then the intrinsic viscosity is measured at 25°C using an automatic viscosity measuring apparatus (Viscotek, made by Malvern Instruments Limited).

Phenolic antioxidant (A)

The polyester layer constituting the multilayer container of the present invention contains a phenolic antioxidant (A).

From the viewpoints of effectively improving the colorlessness and transparency of the recycled polyester, the content of the phenolic antioxidant (A) in the polyester layer is preferably from 0.005 to 0.150 mass%, more preferably from 0.010 to 0.100 mass%, still more preferably from 0.015 to 0.080 mass%, yet still more preferably from 0.015 to 0.050 mass%, yet still more preferably from 0.020 to 0.040 mass%, and yet still more preferably from 0.020 to 0.030 mass% relative to the entire polyester layer.

The phenolic antioxidant (A) is an antioxidant having a phenol structure in which a hydroxyl group is bonded to an aromatic ring in the molecule. The number of phenol structures contained in the molecule is preferably two or more, and more preferably three or more.

Specific examples of the phenolic antioxidant (A) include pentaerythritol tetrakis[3-(3,5-di-tert-butyl-4-hydroxyphenyl)propionate] (Irganox 1010, made by BASF), 3,9-bis[2-[3-(3-t-butyl-4-hydroxy-5-methylphenyl)propionyloxy]-1,1-dimethylethyl]-2,4,8,10-tetraoxaspiro[5.5]undecane (Sumilizer GA-80, made by Sumitomo Chemical Co., Ltd.), octadecyl-3-(3,5-di-tert-butyl-4-hydroxyphenyl)propionate, thiodiethylene bis[3-(3,5-di-tert-butyl-4-hydroxyphenyl)propionate], N,N'-hexane-1,6-diyl bis[3-(3,5-di-tert-butyl-4-hydroxyphenyl propionamide)], 2,4-dimethyl-6-(1-methylpentadecyl)phenol, diethyl [[3,5-bis(1,1-dimethylethyl)-4-hydroxyphenyl]methyl]phosphate, 3,3',3",5,5',5"-hexa-tert-butyl-a,a',a"-(mesitylene-2,4,6-triyl)tri-p-cresol, 4,6-bis(octylthiomethyl)-o-cresol, ethylenebis(oxyethylene)bis[3-(5-tert-butyl-4-hydroxy-m-tolyl)propionate], hexamethylenebis[3-(3,5-di-tert-butyl-4-hydroxyphenyl)propionate], N,N'-hexamethylenebis(3,5-di-t-butyl-4-hydroxy-hydrocinnamamide), 1,3,5-tris(3,5-di-tert-butyl-4-hydroxybenzyl)-1,3,5-triazine-2,4,6(1H, 3H, 5H)-trione, and 2,6-di-tert-butyl-4-(4,6-bis(octylthio)-1,3,5-triazine-2-ylamino)phenol. These may be used singly, or two or more kinds thereof may be used in combination. Among them, pentaerythritol tetrakis[3-(3,5-di-tert-butyl-4-hydroxyphenyl)propionate] (Irganox 1010, made by BASF) is preferable from the viewpoint of effectively improving the colorless transparency of the recycled polyester.

Phosphorus-based antioxidant (B)

The polyester layer constituting the multilayer container of the present invention contains a phosphorus-based antioxidant (B). Note that, the phosphorus-based antioxidant (B) used in the present invention is a compound having a pentaerythritol skeleton and an aromatic ring.

From the viewpoint of effectively improving the colorlessness and transparency of the recycled polyester, the content of the phosphorus-based antioxidant (B) in the polyester layer is preferably from 0.015 to 0.220 mass%, more preferably from 0.030 to 0.150 mass%, still more preferably from 0.045 to 0.150 mass%, yet still more preferably from 0.045 to 0.150 mass%, yet still more preferably from 0.060 to 0.120 mass%, and yet still more preferably from 0.060 to 0.090 mass% relative to the entire polyester layer.

In addition, the total content of the phenolic antioxidant (A) and the phosphorus-based antioxidant (B) in the polyester layer is preferably from 0.04 to 0.22 mass%, more preferably from 0.05 to 0.22 mass%, still more preferably from 0.05 to 0.22 mass%, yet still more preferably from 0.05 to 0.22 mass%, and yet still more preferably from 0.07 to 0.22 mass% relative to the entire polyester layer from the viewpoint of effectively improving the colorlessness and transparency of the recycled polyester, and is yet still more preferably from 0.07 to 0.16 mass%, yet still more preferably from 0.08 to 0.16 mass%, and yet still more preferably from 0.08 to 0.12 mass% particularly from the viewpoint of enhancing the transparency while maintaining the colorlessness of the recycled polyester.

In addition, the mass ratio [(A)/(B)] of the content of the phenolic antioxidant (A) to the content of the phosphorus-based antioxidant (B) is preferably from 1/9 to 6/4, more preferably from 1/9 to 5/5, still more preferably from 2/8 to 5/5, and yet still more preferably from 2/8 to 4/6, from the viewpoint of effectively improving the colorless transparency of the recycled polyester.

The phosphorus-based antioxidant (B) is a compound having a pentaerythritol skeleton and an aromatic ring, and is any compound as long as it has a pentaerythritol skeleton and an aromatic ring in the molecule, but is preferably a compound represented by the following general Formula (1). The compound represented by the following general Formula (1) is bis(substituted phenyl)pentaerythritol diphosphite. The phosphorus-based antioxidant (B) may be used singly, or two or more types thereof may be used in combination.

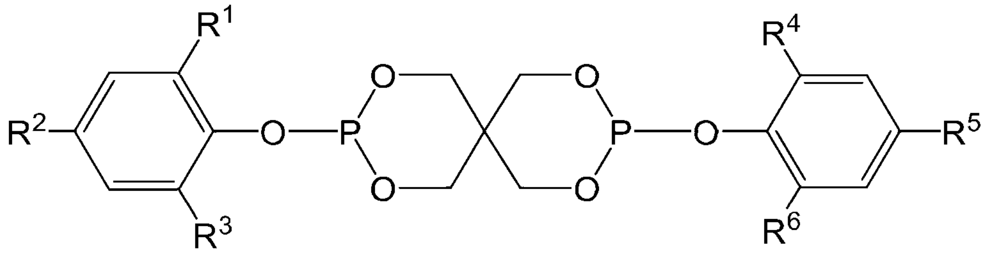

(1)

(where in the formula, $R^1$ to $R^6$ each represent a hydrogen atom or a hydrocarbon group having from 1 to 10 carbon atoms.)

In Formula (1), $R^1$ and $R^4$ may be the same as or different from each other, but are preferably the same as each other. In Formula (1), $R^2$ and $R^5$ may be the same as or different from each other, but are preferably the same as each other. In Formula (1), $R^3$ and $R^6$ may be the same as or different from each other, but are preferably the same as each other.

$R^1$ and $R^4$ each are a hydrogen atom or a hydrocarbon group having from 1 to 10 carbon atoms, preferably an alkyl group having from 1 to 4 carbon atoms, a benzyl group, a phenylethyl group, or a cumyl group, more preferably an alkyl group having 4 carbon atoms or a cumyl group, still more preferably a tert-butyl group or a cumyl group, and yet still more preferably a cumyl group.

$R^2$ and $R^5$ each are a hydrogen atom or a hydrocarbon group having from 1 to 10 carbon atoms, preferably an alkyl group having from 1 to 4 carbon atoms, a benzyl group, a phenylethyl group, or a cumyl group, more preferably an alkyl group having from 1 to 3 carbon atoms or a cumyl group, still more preferably a methyl group or a cumyl group, and yet still more preferably a cumyl group.

$R^3$ and $R^6$ each are a hydrogen atom or a hydrocarbon group having from 1 to 10 carbon atoms, and each are preferably a hydrogen atom, an alkyl group having from 1 to 4 carbon atoms, a benzyl group, a phenylethyl group, or a cumyl group. When $R^1$ and $R^4$ each are a tert-butyl group, $R^3$ and $R^6$ each are preferably a tert-butyl group, and when $R^1$ and $R^4$ each are a cumyl group, $R^3$ and $R^6$ each are preferably a hydrogen atom.

Examples of the phosphorus-based antioxidant (B) include bis(nonylphenyl)pentaerythritol diphosphite, bis(2,4-di-t-butylphenyl)pentaerythritol diphosphite, bis(2,6-di-t-butyl-4-methylphenyl)pentaerythritol diphosphite, and bis(2,4-dicumylphenyl)pentaerythritol diphosphite. Among them, from the viewpoint of effectively improving the colorless transparency of the recycled polyester, bis(2,6-di-t-butyl-4 methylphenyl)pentaerythritol diphosphite and bis(2,4-dicumylphenyl)pentaerythritol diphosphite are preferable, and bis(2,4-dicumylphenyl)pentaerythritol diphosphite is more preferable.

Aldehyde catcher (C)

The polyester layer preferably further contains an aldehyde catcher (C). That is, the multilayer container of the present invention is preferably a multilayer container including a polyester layer containing a polyester resin (X), a phenolic antioxidant (A), a phosphorus-based antioxidant (B), and an aldehyde catcher (C), and a polyamide layer containing a polyamide resin (Y), in which the phosphorus-based antioxidant (B) is a compound having a pentaerythritol skeleton and an aromatic ring.

The aldehyde catcher (C) is a compound having an effect of trapping an aldehyde and suppressing yellowing in the recycling step upon obtaining a recycled polyester resin.

Examples of the aldehyde catcher (C) include a compound having an ability to suppress yellowing of a polyester resin and containing an amino group, specifically, the aldehyde catcher (C) is preferably at least one compound selected from the group consisting of aminobenzamide, aminobenzoic acid, diaminobenzoic acid, and nylon 6I/6T, and more preferably at least one compound selected from the group consisting of anthranilamide, anthranilic acid, and nylon 6I/6T.

The amino group in the aminobenzamide may occupy any one of substituent positions 2 to 4, but preferably occupies substituent position 2 or 3, and the aminobenzamide is preferably anthranilamide (2-aminobenzamide).

The amino group in the aminobenzoic acid may occupy any substituent position 2, 3, or 4, but preferably occupies substituent position 2 or 3, more preferably occupies substituent position 2, and the aminobenzoic acid is more preferably anthranilic acid (2-aminobenzoic acid).

The diaminobenzoic acid may be 2,3-distributed, 2,4-distributed, or 3,4-distributed, and the diaminobenzoic acid is preferably 3,4-diaminobenzoic acid.

Nylon 6I/6T is a hexamethylenediamine-isophthalic acid-terephthalic acid copolymerized polyamide, and is a hexamethylene isophthalamide/hexamethylene terephthalamide copolymer. As the nylon 6I/6T, a commercially available product may be used, and examples thereof include Selar (trade name) PA 3426 (made by DuPont) and NOVAMID X21 (made by DSM).

The weight average molecular weight of nylon 6I/6T is preferably from 10,000 to 50,000, more preferably from 15,000 to 45,000, and still more preferably from 20,000 to 40,000. The weight average molecular weight is a value

measured by gel permeation chromatography and converted calibrated with polystyrene. When the weight average molecular weight of the nylon 6I/6T is within the range mentioned above, the nylon 6I/6T is excellent in miscibility with the polyester resin, and, when it is used to form a container, its elusion into the content in the container can be suppressed, and yellowing is effectively suppressed.

The concentration of the amino end group of nylon 6I/6T is preferably from 50 to 350 μmol/g, more preferably from 100 to 300 μmol/g, and still more preferably from 150 to 250 μmol/g. When the concentration of the amino end group of nylon 6I/6T is within the range mentioned above, the effect of suppressing yellowing of the recycled polyester is excellent.

The concentration of the amino end group is determined by neutralization titration, in which precisely weighed nylon 6I/6T is put into a solution of phenol/ethanol = 4/1 in volume at 20 to 30°C and stirred and mixed, after complete dissolution, while stirring it, the inner wall of the container is washed with 5 mL of methanol, and the solution is neutralized by titration using 0.01 mol/L hydrochloric acid aqueous solution.

Other than aminobenzamide, aminobenzoic acid, diaminobenzoic acid, and nylon 6I/6T described above, other examples of the aldehyde catcher (C) include salicylamide, salicylanilide, o-phenylenediamine, 1,8-diaminonaphthalene, o-mercaptobenzamide, N-acetylglycinamide, malonamide, 3-mercapto-1,2-propanediol, histidine, tryptophan, 4-amino-3-hydroxybenzoic acid, biuret, 2,3-diaminopyridine, 1,2-diaminoanthraquinone, dianilinoethane, allantoin, and 2-amino-2-methyl-1,3-propanediol.

The aldehyde catcher (C) may be used singly, or two or more kinds thereof may be used in combination.

The aldehyde catcher (C) is preferably at least one compound selected from the group consisting of anthranilamide and nylon 6I/6T, more preferably anthranilamide or nylon 6I/6T, and still more preferably anthranilamide.

The content of the aldehyde catcher (C) in the polyester layer is preferably from 0.003 to 0.150 mass%, more preferably from 0.005 to 0.150 mass%, still more preferably from 0.005 to 0.120 mass%, still more preferably from 0.005 to 0.100 mass%, yet still more preferably from 0.005 to 0.080 mass%, yet still more preferably from 0.010 to 0.070 mass%, and yet still more preferably from 0.020 to 0.060 mass% relative to the entire polyester layer from the viewpoint of effectively suppressing yellowing.

In addition, the total content of the phenolic antioxidant (A), the phosphorus-based antioxidant (B), and the aldehyde catcher (C) in the polyester layer is from 0.05 to 0.22 mass%, and preferably from 0.07 to 0.22 mass% relative to the entire polyester layer from the viewpoint of effectively suppressing yellowing, and is more preferably from 0.07 to 0.16 mass%, still more preferably from 0.08 to 0.16 mass%, and yet still more preferably from 0.08 to 0.12 mass% particularly from the viewpoint of enhancing the transparency while maintaining the colorlessness of the recycled polyester.

In light of the above, as the multilayer container of the present invention, there is preferably used a multilayer container including: a polyester layer containing a polyester resin (X), a phenolic antioxidant (A), a phosphorus-based antioxidant (B), and an aldehyde catcher (C); and a polyamide layer containing a polyamide resin (Y), wherein the phosphorus-based antioxidant (B) is a compound having a pentaerythritol skeleton and an aromatic ring, the aldehyde catcher (C) is anthranilamide, the total content of the phenolic antioxidant (A), the phosphorus-based antioxidant (B), and the aldehyde catcher (C) in the polyester layer is from 0.05 to 0.22 mass%, and the content of the aldehyde catcher (C) in the polyester layer is from 0.005 to 0.150 mass%, the mass ratio [(A)/(B)] of the content of the phenolic antioxidant (A) to the content of the phosphorus-based antioxidant (B) is from 2/8 to 5/5.

Other components

The polyester layer may contain other components. Examples of the other components include a heat stabilizer, a light stabilizer, a moisture-proof agent, a waterproof agent, a lubricant, and a spreading agent.

A resin other than the main component polyester resin (X) may be contained in the polyester layer within a range not impairing the effects of the present invention. The content of the polyester resin (X) is preferably from 80 to 100 mass% and more preferably from 90 to 100 mass% relative to the resin amount of the entire polyester layer, and the polyester layer may be composed only of the polyester resin (X).

Polyester resin composition used for polyester layer

The polyester layer constituting the multilayer container of the present invention contains a polyester resin (X), a phenolic antioxidant (A), and a phosphorus-based antioxidant (B), and contains an optional component such as an aldehyde catcher (C). Therefore, a polyester resin composition containing these components is preferably used in forming the multilayer container. The method for producing the polyester resin composition is not limited, but the polyester resin composition is preferably produced by the following method.

The polyester resin composition is preferably obtained by melt-mixing of the polyester resin (X), the phenolic antioxidant (A), the phosphorus-based antioxidant (B), the aldehyde catcher (C) and the like.

The phenolic antioxidant (A), the phosphorus-based antioxidant (B), the aldehyde catcher (C), and other components (additives) may be directly added to the resin, and melt-mixed, or they may be dissolved in a liquid

component to form an additive solution, which is then added to the resin, and melt-mixed. It is preferable to prepare a solution because the measurement and addition are facilitated. Also, it is preferable because a solution can be added using a dosing system or the like. The additive solution may be added at the time when the masterbatch and the polyester resin (X) are dry blended, or may be added after the polyester resin (X) has been melted.

The amount of the liquid component used in the additive solution is preferably from 0.01 to 1 mass% and more preferably from 0.01 to 0.5 mass% relative to the resin (e.g., polyester resin (X)) constituting the polyester layer. The amount of liquid component used in the additive solution is preferably from 0.01 to 1 mass%, more preferably from 0.01 to 0.5 mass% relative to the polyester resin (X) constituting the polyester layer.

The liquid component is preferably a liquid resin or a liquid oily component.

Examples of the liquid resin include: epoxy-based resins such as epoxidized soybean oil and epoxidized linseed oil; fatty acid polyester resins; polyalkylene glycol resins; polyether ester resins; and tributyl acetyl citrate.

Examples of the liquid oily component include: vegetable oils such as olive oil, castor oil, jojoba oil, macadamia nut oil, Rosa canina fruit oil, cacao butter and lanolin; animal oils such as horse oil, turtle oil, wild boar oil, mink oil, and shark oil; hydrocarbon oils such as vaseline, liquid paraffin, isodecane, isododecane, octyldodecyl, diisostearyl malate, and hydrogenated polyisobutene; ester oils such as isotridecyl isononanoate, isopropyl isostearate, neopentyl glycol dicaprate, isotridecyl isononanoate, glyceryl diisostearate, glyceryl triisostearate, diisostearyl malate, octyldodecanol, and di(phytosteryl/2-octyldodecyl)-N-lauroyl-L-glutamate; silicone oils such as dimethylpolysiloxane and phenylmethylpolysiloxane; dimer acid esters, dimer diol derivatives, cholesterol fatty acid esters, phytosterol fatty acid esters, polyglycerol fatty acid esters, pentaerythritol fatty acid esters, and glyceryltri(2-ethylhexanoate).

The method for producing the additive solution is not particularly limited, and for example, a liquid component and various additives are added, mixed using a Henschel mixer, a tumbler, a disper, or the like, and dispersed using a Silverson mixer (made by Silverson), whereby the additive solution can be obtained. As the dispersing apparatus, other than the apparatuses mentioned above, any apparatus such as a kneader, a roll mill, a ball mill, or a sand mill can be used.

Examples of the melt-mixing method include melt blending (melt-kneading). In producing a multilayer container to be described later, the polyester resin (X), the phenolic antioxidant (A), the phosphorus-based antioxidant (B), the aldehyde catcher (C), and the like may be dry blended beforehand, and then melt-mixed in the step of obtaining a multilayer preform.

Examples of the melt blending include a masterbatch method and a full compound method, and the masterbatch method is preferable from the viewpoint of preventing deterioration of the resin and the antioxidant.

The masterbatch method is a method in which a small amount of a resin, a phenolic antioxidant (A), a phosphorus-based antioxidant (B), an aldehyde catcher (C), and the like are mixed to form a masterbatch, and then the masterbatch is mixed with the remaining polyester resin (X).

From the viewpoint of compatibility with the polyester resin (X), the resin used in the masterbatch is preferably the polyester resin (X), and more preferably the same resin as the remaining polyester resin (X).

The amount of the resin used in the masterbatch is preferably from 1 to 20 mass%, more preferably from 3 to 15 mass% relative to the resin amount of the entire polyester resin composition.

As a method for obtaining a masterbatch, when kneading the resin, the phenolic antioxidant (A), the phosphorus-based antioxidant (B), the aldehyde catcher (C), and the like, from the viewpoint of thorough mixing, the kneading temperature (°C) is preferably from Tm + 5 to Tm + 60, more preferably from Tm + 10 to Tm + 50, still more preferably from Tm + 15 to Tm + 40, wherein Tm is the melting point of the resin used in the masterbatch. Specifically, the temperature is yet still more preferably from 245 to 300°C, yet still more preferably from 250 to 290°C, and yet still more preferably from 255 to 280°C. The kneading time is preferably from 10 to 600 seconds, more preferably from 20 to 400 seconds, and still more preferably from 30 to 300 seconds from the viewpoint of thorough mixing. Examples of the apparatus used for kneading include an open type mixing roll, a non-open type Banbury mixer, a kneader, and a continuous kneader (such as a single-screw kneader, a twin-screw kneader, and a multi-screw kneader).

Examples of a method of melt-mixing the masterbatch and the remaining of the polyester resin (X) include melt blending (melt-kneading). When producing a multilayer container to be described later, the masterbatch and the remaining polyester resin (X) may be dry blended beforehand, and then melt-mixed in the step of obtaining a multilayer preform.

The full compound method is a method in which the entire amount of the polyester resin (X) used in the resin composition, the phenolic antioxidant (A), the phosphorus-based antioxidant (B), the aldehyde catcher (C), and the like are kneaded and mixed.

The kneading temperature is preferably from 255 to 310°C, more preferably from 265 to 300°C, and still more preferably from 270 to 290°C from the viewpoint of thorough mixing. The kneading time is preferably from 10 to 600 seconds, more preferably from 20 to 400 seconds, and still more preferably from 30 to 300 seconds from the viewpoint of thorough mixing. Examples of the apparatus used for kneading include an open type mixing roll, a non-open type Banbury mixer, a kneader, and a continuous kneader (such as a single-screw kneader, a twin-screw kneader, and a multi-screw kneader).

Polyamide Layer

The polyamide layer contains a polyamide resin (Y).

The content of the polyamide resin (Y) in the polyamide layer is preferably from 70 to 100 mass% relative to the total amount of the polyamide layer, and from the viewpoints of gas barrier properties and suppression of yellowing of the recycled polyester, the content is more preferably from 90 to 100 mass%, still more preferably from 95 to 100 mass%, and yet still more preferably from 99 to 100 mass%. The polyamide layer may be composed of the polyamide resin (Y), or may be composed only of the polyamide resin (Y).

The content of the polyamide resin (Y) contained in the polyamide layer is preferably from 0.05 to 10.0 mass% relative to the total amount of all the polyamide layers and all the polyester layers, and from the viewpoints of gas barrier properties and suppression of yellowing of the recycled polyester, the content is more preferably from 0.5 to 9.0 mass%, still more preferably from 1.0 to 8.0 mass%, and yet still more preferably from 3.0 to 7.0 mass%.

The content of the polyamide layer is preferably from 0.05 to 10.0 mass% relative to the total amount of all the polyamide layers and all the polyester layers, and from the viewpoints of gas barrier properties and suppression of yellowing of the recycled polyester, the content is more preferably from 0.5 to 9.0 mass%, still more preferably from 1.0 to 8.0 mass%, and yet still more preferably from 3.0 to 7.0 mass%.

Polyamide resin (Y)

Examples of the polyamide resin (Y) include xylylene group-containing polyamide resins, nylon 6, nylon 66, nylon 666, nylon 610, nylon 11, nylon 12, and mixtures thereof. Among them, a xylylene group-containing polyamide resin is preferable because it can improve gas barrier performance and is easily separated from a polyester layer during recycling. The xylylene group-containing polyamide resin is preferably a polyamide resin containing a xylylenediamine-derived structural unit.

The xylylene group-containing polyamide resin is a polycondensation product of a diamine containing xylylenediamine and a dicarboxylic acid, and includes a xylylenediamine-derived structural unit and a dicarboxylic acid-derived structural unit. The xylylene group-containing polyamide resin preferably contains the xylylenediamine-derived structural unit in an amount of 50 mol% or more, more preferably 70 mol% or more, still more preferably from 80 to 100 mol%, and yet still more preferably from 90 to 100 mol% of the diamine-derived structural unit (diamine unit).

The xylylenediamine is preferably meta-xylylenediamine, para-xylylenediamine, or both of them, more preferably meta-xylylenediamine. The diamine unit constituting the xylylene group-containing polyamide resin preferably contains the meta-xylylenediamine-derived structural unit in an amount of 50 mol% or more, more preferably 70 mol% or more, still more preferably from 80 to 100 mol%, and yet still more preferably from 90 to 100 mol%. When the amount of the meta-xylylenediamine-derived structural unit in the diamine unit is within the range mentioned above, the polyamide resin is further improved in gas barrier properties.

The diamine unit in the xylylene group-containing polyamide resin may be composed only of the xylylenediamine-derived structural unit, or may contain a structural unit derived from a diamine other than xylylenediamine. Here, examples of the diamine other than xylylenediamine include: aliphatic diamines having a linear or branched structure, such as ethylenediamine, tetramethylenediamine, pentamethylenediamine, 2-methylpentanediamine, hexamethylenediamine, heptamethylenediamine, octamethylenediamine, nonamethylenediamine, decamethylenediamine, dodecamethylenediamine, 2,2,4-trimethyl-hexamethylenediamine, and 2,4,4-trimethyl-hexamethylenediamine; alicyclic diamines such as 1,3-bis(aminomethyl)cyclohexane, 1,4-bis(aminomethyl)cyclohexane, 1,3-diaminocyclohexane, 1,4-diaminocyclohexane, bis(4-aminocyclohexyl)methane, 2,2-bis(4-aminocyclohexyl)propane, bis(aminomethyl)decalin, and bis(aminomethyl)tricyclodecane; and diamines having an aromatic ring, such as bis(4-aminophenyl)ether, paraphenylenediamine, and bis(aminomethyl)naphthalene.

As the compound capable of constituting the dicarboxylic acid unit in the xylylene group-containing polyamide resin, examples thereof include: $\alpha,\omega$-linear aliphatic dicarboxylic acids having from 4 to 20 carbon atoms, such as succinic acid, glutaric acid, adipic acid, pimelic acid, suberic acid, azelaic acid, sebacic acid, undecanedioic acid, and dodecanedioic acid; alicyclic dicarboxylic acids such as 1,4-cyclohexanedicarboxylic acid; other aliphatic dicarboxylic acids such as dimer acids; and aromatic dicarboxylic acids such as terephthalic acid, isophthalic acid, orthophthalic acid, xylylenedicarboxylic acid, and naphthalenedicarboxylic acid, and an $\alpha,\omega$-linear aliphatic dicarboxylic acid having from 4 to 20 carbon atoms is preferable, adipic acid and sebacic acid are more preferable, and adipic acid is still more preferable from the viewpoint of improving barrier performance.

The xylylene group-containing polyamide resin preferably contains the adipic acid-derived structural unit in an amount of 50 mol% or more, more preferably 70 mol% or more, still more preferably from 80 to 100 mol%, and yet still more preferably from 90 to 100 mol% of the dicarboxylic acid-derived structural unit (dicarboxylic acid unit).

That is, the polyamide resin (Y) contains structural units derived from a diamine and structural units derived from a dicarboxylic acid, preferably 50 mol% or more of the structural units derived from a diamine being structural units derived

from xylylenediamine, and 50 mol% or more of the structural units derived from a dicarboxylic acid being structural units derived from adipic acid; more preferably 80 mol% or more of the structural units derived from a diamine being structural units derived from xylylenediamine, and 80 mol% or more of the structural units derived from a dicarboxylic acid being structural units derived from adipic acid; and still more preferably 90 mol% or more of the structural units derived from a diamine being structural units derived from xylylenediamine, and 90 mol% or more of the structural units derived from a dicarboxylic acid being structural units derived from adipic acid.

Xylylenediamine is preferably meta-xylylenediamine.

The remaining dicarboxylic acid unit other than adipic acid is preferably a structural unit derived from an $\alpha,\omega$-linear aliphatic dicarboxylic acid having from 4 to 20 carbon atoms.

Furthermore, examples of the preferred xylylene group-containing polyamide resin include: polyamide resins in which 70 mol% or more of the diamine unit is a structural unit derived from xylylenediamine (preferably meta-xylylenediamine), 70 to 99 mol% of the dicarboxylic acid unit is a structural unit derived from adipic acid, and 1 to 30 mol% of the dicarboxylic acid unit is a structural unit derived from isophthalic acid. The polyamide resin is preferably a polyamide resin in which 80 mol% or more of the diamine unit are structural units derived from xylylenediamine (preferably meta-xylylenediamine), 80 to 99 mol% of the dicarboxylic acid unit are structural units derived from adipic acid, and 1 to 20 mol% of the diamine unit are structural units derived from isophthalic acid, and more preferably a polyamide resin in which 90 mol% or more of the diamine unit are structural units derived from xylylenediamine (preferably meta-xylylenediamine), 80 to 99 mol% of the dicarboxylic acid unit are structural units derived from adipic acid, and 1 to 20 mol% of the dicarboxylic acid unit are structural units derived from isophthalic acid.

When an isophthalic acid unit is added as a dicarboxylic acid unit, the melting point is lowered and the molding processing temperature can be lowered, and hence thermal degradation during molding can be suppressed, and the crystallization time is delayed and thereby improving the stretching moldability.

Other than the above-described diamine and dicarboxylic acid, as components constituting the xylylene group-containing polyamide resin, there can be used lactams such as $\varepsilon$-caprolactam and laurolactam; aliphatic aminocarboxylic acids such as aminocaproic acid and aminoundecanoic acid; and aromatic aminocarboxylic acids such as p-aminomethylbenzoic acid within a range not impairing the effects of the present invention.

The xylylene group-containing polyamide resin is preferably produced by a polycondensation reaction in a molten state (hereinafter, described as "melt polycondensation" in some cases). For example, the xylylene group-containing polyamide resin is preferably produced by a method in which a nylon salt composed of a diamine and a dicarboxylic acid is heated in the presence of water by a pressurization method and polymerization is carried out in a molten state at the same time water is removed. Alternatively, the xylylene group-containing polyamide resin may be produced by a method in which a diamine is directly added to a dicarboxylic acid in a molten state and polycondensation is carried out under normal pressure. In this case, in order to keep the reaction system in a homogeneous liquid state, polycondensation is preferably allowed to proceed by continuously adding the diamine to the dicarboxylic acid at the same time raising the temperature of the reaction system such that the reaction temperature does go down below the melting points of the resulting oligoamides and polyamides. If necessary, the xylylene group-containing polyamide obtained by melt polycondensation can be allowed to further undergo solid-phase polymerization and thereby increasing the molecular weight.

The xylylene group-containing polyamide resin preferably undergoes polycondensation in the presence of a phosphorus atom-containing compound. When the xylylene group-containing polyamide resin undergoes polycondensation in the presence of the phosphorus atom-containing compound, processing stability during melt-molding is enhanced, and coloring is more likely to be suppressed.

As the phosphorus atom-containing compound, a hypophosphorous acid compound and a phosphorous acid compound are preferable, and a hypophosphorous acid compound is more preferable.

The phosphorus atom-containing compound is preferably organic metal salts, and among these, alkali metal salts are more preferable.

Examples of the hypophosphorous acid compound include hypophosphorous acid, a hypophosphorous acid metal salt, a phenylphosphonous acid metal salt, ethyl hypophosphite, dimethylphosphinic acid, phenyl methyl phosphinic acid, phenylphosphonous acid, and ethyl phenylphosphonite from the viewpoint of promoting the polymerization reaction and from the viewpoint of preventing coloring, and a hypophosphorous acid metal salt is preferable.

Examples of the hypophosphorous acid metal salt include sodium hypophosphite, potassium hypophosphite, lithium hypophosphite, and calcium hypophosphite, and sodium hypophosphite is more preferable.

Examples of the phenylphosphonous acid metal salt include sodium phenylphosphonite, potassium phenylphosphonite, and lithium phenylphosphonite.

Examples of the phosphorous acid compound include phosphorous acid, pyrophosphorous acid, phosphorous acid metal salt, phenylphosphonic acid metal salt, triethyl phosphite, triphenyl phosphite, ethylphosphonic acid, phenylphosphonic acid, and diethyl phenylphosphonate.

Examples of the phosphorous acid metal salt include sodium hydrogen phosphite, sodium phosphite, potassium

phosphite, and calcium phosphite.

Examples of the ethylphosphonic acid metal salt include sodium ethyl phosphonate and potassium ethyl phosphonate.

Examples of the phenylphosphonic acid metal salt include sodium phenylphosphonate, potassium phenylphosphonate, and lithium phenylphosphonate.

The phosphorus atom-containing compound may be used singly, or two or more kinds thereof may be used in combination.

The polycondensation of the xylylene group-containing polyamide resin is preferably performed in the presence of a phosphorus atom-containing compound and an alkali metal compound. When a large amount of the phosphorus atom-containing compound is used, the polyamide resin is liable to be gelled. Therefore, an alkali metal compound is preferably allowed to coexist from the viewpoint of adjusting the amidation reaction rate.

Examples of the alkali metal compound include alkali metal hydroxides and alkali metal acetates. Examples of the alkali metal hydroxide include lithium hydroxide, sodium hydroxide, potassium hydroxide, rubidium hydroxide, and cesium hydroxide, and examples of the alkali metal acetate include lithium acetate, sodium acetate, potassium acetate, rubidium acetate, and cesium acetate.

When an alkali metal compound is used in polycondensation of the polyamide resin, the amount of the alkali metal compound used, as a value of the number of moles of the alkali metal compound divided by the number of moles of the phosphorus atom-containing compound, is preferably from 0.5 to 1, more preferably from 0.55 to 0.95, and still more preferably from 0.6 to 0.9 from the viewpoint of suppressing gel formation.

The number average molecular weight of the polyamide resin is appropriately selected depending on the intended use of the multilayer container and the molding method, but is preferably from 10,000 to 60,000 and more preferably from 11,000 to 50,000 from the viewpoints of moldability and strength of the multilayer container.

Note that the number average molecular weight of the polyamide resin is calculated from the following formula (2).

$$\text{The number average molecular weight} = 2 \times 1{,}000{,}000/([COOH] + [NH_2])....(2)$$

(where [COOH] represents the concentration of the end carboxyl group (μmol/g) in the polyamide resin, and [$NH_2$] represents the concentration of the terminal amino group (μmol/g) in the polyamide resin.)

Here, as the concentration of the end carboxyl group is used a value calculated from the neutralization titration of a solution of polyamide dissolved in benzyl alcohol using an aqueous sodium hydroxide solution.

In the present invention, the concentration of the terminal amino group of the polyamide resin (Y) is preferably 50 μmol/g or less, more preferably 45 μmol/g or less, still more preferably 40 μmol/g or less, even more preferably 30 μmol/g or less, even more preferably 20 μmol/g or less from the viewpoint of suppressing yellowing of the recycled polyester.

The concentration of the terminal amino group of the polyamide resin (Y) is determined by neutralization titration, in which precisely weighed polyamide resin is put into a solution of phenol/ethanol = 4/1 in volume at 20 to 30°C and stirred and mixed, after complete dissolution, while stirring it, the inner wall of the container is washed with 5 mL of methanol, and the solution is neutralized by titration using 0.01 mol/L hydrochloric acid aqueous solution.

The method for adjusting the concentration of the terminal amino group of the polyamide resin (Y) is not particularly limited, but the concentration of the terminal amino group can be kept low by a method in which a polycondensation reaction is performed by adjusting the charged ratio (molar ratio) between the diamine and the dicarboxylic acid, a method in which a monocarboxylic acid that caps an amino group is fed together with the diamine and the dicarboxylic acid to perform a polycondensation reaction, a method in which a polycondensation reaction is performed and then the terminal amino group is allowed to react with a carboxylic acid that caps an amino group, or the like.

Other components

The polyamide layer may contain other components. Examples of the other components include a heat stabilizer, a light stabilizer, a moisture-proof agent, a waterproof agent, a lubricant, and a spreading agent.

A resin other than the main component polyamide resin (Y) may be contained in the polyamide layer within a range not impairing the effects of the present invention.

Structure and Characteristics of Multilayer Container

The multilayer container of the present invention is a multilayer container including: a polyester layer containing a polyester resin (X), a phenolic antioxidant (A), and a phosphorus-based antioxidant (B); or a polyester layer containing a polyester resin (X), a phenolic antioxidant (A), a phosphorus-based antioxidant (B), and an aldehyde catcher (C); and a

polyamide layer containing a polyamide resin (Y), in which the total content of the phenolic antioxidant (A), the phosphorus-based antioxidant (B), and the aldehyde catcher (C) in the polyester layer is from 0.05 to 0.22 mass%, and the phosphorus-based antioxidant (B) is a compound having a pentaerythritol skeleton and an aromatic ring.

The multilayer container of the present invention may contain a resin layer other than the polyester layer and the polyamide layer, but from the viewpoint of facilitating the separation in recycling and from the viewpoint of improving the color tone of the container and the recovered polyester, the resin layer other than the polyester layer and the polyamide layer is preferably contained in a small amount, and the resin layer other than the polyester layer and the polyamide layer is preferably not contained substantially. In addition, an adhesive layer made of an adhesive and/or an inorganic layer made of an inorganic substance may be provided, but from the viewpoint of facilitating separation in recycling and from the viewpoint of improving an effect of suppressing yellowing, the content of the adhesive layer and/or the inorganic layer is preferably small, and the adhesive layer and/or the inorganic layer is preferably not contained substantially.

The multilayer container of the present invention has a multilayer structure with two or more layers, preferably has a two-layer to five-layer structure, more preferably has a three- to five-layer structure, still more preferably has a three-layer structure or a five-layer structure, and yet still more preferably has a three-layer structure.

The outermost layer of the multilayer container of the present invention is preferably a polyester layer. Moreover, the innermost layer is also preferably a polyester layer, and it is more preferable that both the outermost layer and the innermost layer are polyester layers.

When the outermost layer is a polyester layer, the multilayer container is excellent in impact resistance, appearance, and design.

Therefore, as the structure of the multilayer container, the multilayer container preferably has a two- to five-layer structure in which the outermost layer is a polyester layer, and the multilayer container more preferably has a three- to five-layer structure in which the outermost layer and the innermost layer each are a polyester layer.

In the case of a two-layer structure, the order from the innermost layer is preferably polyamide layer/polyester layer, in the case of a three-layer structure, the order from the innermost layer is preferably polyester layer/polyamide layer/polyester layer, and in the case of a five-layer structure, the order from the innermost layer is preferably polyester layer/polyamide layer/polyester layer/polyamide layer/polyester layer.

The multilayer container of the present invention is preferably a hollow container, and when the multilayer container is a hollow container, at least a body thereof has a multilayer structure. In the body, a ratio of a thickness (W) of the polyester layer to a thickness (S) of the polyamide layer (thickness ratio: W/S) is preferably 2.5 or more and 200 or less. Note that, the thickness of the polyester layer means an average thickness, and in the case where a plurality of polyester layers are contained in the body, the thicknesses of the plurality of polyester layers are averaged to determine an average thickness per layer. The thickness of the polyamide layer is determined in the same manner.

The thickness ratio W/S of 2.5 or more is preferable because of facilitating the separation of the polyamide resin from the polyester resin in a separation step, particularly in air elutriation or specific gravity separation in a method for producing recycled polyester. When the thickness ratio W/S is 200 or less, the hollow container is excellent in gas barrier properties, and contents therein can be stored for a long period of time.

From the viewpoint of improving the gas barrier properties of the hollow container while improving the separability in the separation step, the thickness ratio (W/S) is more preferably from 3 to 50, still more preferably from 4 to 15.

When the multilayer container is a hollow container, the total thickness of the body (i.e., the total thickness of all the layers of the body) of the hollow container is preferably from 100 μm to 5 mm, more preferably from 150 μm to 3 mm, and still more preferably from 200 μm to 2 mm. The thickness (W) of each polyester layer is preferably from 30 μm to 2 mm, more preferably from 40 μm to 1 mm, and still more preferably from 50 μm to 500 μm. The thickness (S) of each polyamide layer is preferably from 1 to 200 μm, more preferably from 3 to 100 μm, and still more preferably from 8 to 50 μm. In the present invention, the thickness of the polyamide layer is adjusted to be within this range, whereby the polyamide layer is easily separated from the polyester in the separation step while the gas barrier properties are ensured.

When the multilayer container of the present invention is a hollow container, the hollow container is more preferably a liquid packaging container that is used by filling the inside of the hollow container with a liquid, and still more preferably a beverage packaging container. Examples of the liquid to be filled inside include beverages, liquid seasonings, chemical products, pharmaceuticals, and detergents, and preferable are beverages whose deterioration due to oxygen can be effectively prevented by the multilayer container of the present invention.

Examples of the beverage include water, carbonated water, oxygen water, hydrogen water, milk, dairy products, juice, coffee, coffee beverages, carbonated soft drinks, teas, and alcoholic beverages.

Examples of the liquid seasoning include sauce, soy sauce, syrup, sweet sake, dressing, and the like.

Examples of the chemical products include pesticides and insecticides.

Method for Producing Multilayer Container

The method for producing a multilayer container of the present invention is not particularly limited, as long as it is a

method for producing a multilayer container including a polyester layer containing a polyester resin (X), a phenolic antioxidant (A) and a phosphorus-based antioxidant (B) and a polyamide layer containing a polyamide resin (Y), but it is preferably a production method including the following step 1 and the following step 2.

That is, the method for producing a multilayer container of the present invention is preferably a production method including the following steps 1 and 2, which is a method for producing a multilayer container including a polyester layer containing a polyester resin (X), a phenolic antioxidant (A), and a phosphorus-based antioxidant (B), and a polyamide layer containing a polyamide resin (Y).

Step 1: A step of co-injection molding a polyamide resin (Y) used for a polyamide layer, a polyamide resin composition containing a polyamide resin (Y), or a polyamide resin mixture containing a polyamide resin (Y); and a polyester resin composition containing a polyester resin (X), a phenolic antioxidant (A), and a phosphorus-based antioxidant (B), or a polyester resin mixture containing a polyester resin (X), a phenolic antioxidant (A), and a phosphorus-based antioxidant (B) to obtain a multilayer preform

Step 2: A step of blow molding the multilayer preform

Step 1 (Step to obtain multilayer preform)

In step 1, the polyamide resin (Y), the polyamide resin composition or the polyamide resin mixture, and the polyester resin composition or the polyester resin mixture are subjected to co-injection molding to obtain a multilayer preform.

The polyamide resin composition is a composition in which the polyamide resin (Y) is contained as a main component and which contains a resin other than the polyamide resin (Y), other components etc., described in the "Polyamide Layer" section.

The polyester resin composition is a composition in which the polyester resin (X) is contained as a main component and which contains the phenolic antioxidant (A) and the phosphorus-based antioxidant (B), and may contain the aldehyde catcher (C), a resin other than the polyester resin (X), other components etc., described in the "Polyester Layer" section.

Note that, the polyamide resin mixture refers to a dry blended mixture of the polyamide resin (Y), a resin other than the polyamide resin (Y), and other components etc., or a dry blended mixture of a masterbatch containing other components etc., and the remaining polyamide resin (Y). The respective components are melt-mixed in this step, whereby the polyamide resin mixture is formed into the polyamide resin composition.

The polyester resin mixture means a dry blended mixture of the polyester resin (X), the phenolic antioxidant (A), the phosphorus-based antioxidant (B), the aldehyde catcher (C), a resin other than the polyester resin (X), and other components etc., or a dry blended mixture of a masterbatch containing the phenolic antioxidant (A), the phosphorus-based antioxidant (B), the aldehyde catcher (C), a resin other than the polyester resin (X), and other components etc., and the remaining polyester resin (X). The respective components are melt-mixed in this step, whereby the polyester resin mixture is formed into the polyester resin composition.

In co-injection molding, the polyester resin (composition, mixture) and the polyamide resin (composition, mixture) are each extruded into a mold and are subjected to co-injection molding to form a multilayer preform.

Step 2 (Step of blow molding)

In step 2, the multilayer preform is subjected to blow molding.

In the method for producing a multilayer container of the present invention, the multilayer preform (multilayer parison) obtained in step 1 is preferably molded by stretch blow.

Among them, in step 2, the multilayer preform obtained by co-injection molding is preferably subjected to stretch blow molding, and the multilayer preform obtained by co-injection molding is more preferably subjected to biaxial stretch blow molding. Note that, preferable conditions for the biaxial stretch blow molding are that the preform heating temperature is set to be in a range from 95 to 110°C, the primary blow pressure is set to be in a range from 0.5 to 1.2 MPa, and the secondary blow pressure is set to be in a range from 2.0 to 2.6 MPa, whereby occurrence of uneven thickness and heterogeneity in stretching can be suppressed, and a multilayer container with excellent strength can be obtained.

Method for Producing Recycled Polyester

The method for producing a recycled polyester of the present invention is a method for producing a recycled polyester including a step of recovering polyester from the multilayer container.

Hereinafter, a method for producing the recycled polyester of the present invention will be described in detail.

In the present production method, as the multilayer container, post-consumer items are usually used, but unused multilayer containers may also be used. The post-consumer multilayer containers include items that were recovered after

once distributed on the market.

In the present production method, first, when a lid is attached to the multilayer container, the lid is preferably removed from the multilayer container.

Next, the container is milled, and, if necessary, a separation for selectively taking out polyester is implemented, whereby the polyester is preferably recovered as recycled polyester (Recovery step).

In the recovery step, the container or the milled product is preferably washed with an alkaline aqueous solution (Washing step).

Next, if necessary, the polyester is granulated and formed into pellets (Granulation step).

Furthermore, if necessary, the polyester is subjected to crystallization and solid-phase polymerization (Crystallization/Solid-phase polymerization step).

Each step is described below.

Washing Step

In the method for producing a recycled polyester of the present invention, the multilayer container or the milled product thereof is preferably washed using an alkaline aqueous solution and polyester is recovered.

Washing using an alkaline aqueous solution enables to effectively remove not only contents stored in the multilayer container but also an adhesive and the like.

The washing using an alkaline aqueous solution may be carried out for the containers as they are, may be carried out at the same time as milling, may be carried out after milling, or may be carried out after polyester and polyamide resin have been separated from each other. Washing may be performed more than once. Washing is preferably implemented after milling, but as a matter of convenience, the washing step will be described before the description of the recovery step to be described later.

The solvent of the alkaline aqueous solution used for washing the multilayer container or the milled product thereof is water from the aspects of washing efficiency and cost. Other than water, an aqueous organic solvent may be contained. Examples of the aqueous organic solvent include lower alcohols such as methanol, ethanol, and isopropyl alcohol, and diols.

The pH of the alkaline aqueous solution is preferably 8 or more, more preferably 10 or more, and still more preferably 12 or more. The upper limit is not limited, but is preferably 14 or less.

The alkaline aqueous solution contains an alkaline substance in addition to the solvent mentioned above. The alkaline substance is preferably at least one selected from the group consisting of alkali metal hydroxides and alkaline earth metal hydroxides, and alkali metal hydroxides are more preferable from the aspects of washing efficiency and cost.

Examples of the alkali metal hydroxides include sodium hydroxide, potassium hydroxide, and lithium hydroxide, and from the aspects of washing efficiency and cost, at least one selected from the group consisting of sodium hydroxide and potassium hydroxide is preferable, and sodium hydroxide is more preferable.

The content of the alkaline substance is preferably from 0.1 to 10 mass%, more preferably from 0.5 to 8 mass%, and still more preferably from 1 to 5 mass% relative to the total amount of the alkaline aqueous solution.

A washing apparatus may use any method, but a container equipped with a stirrer is preferably used, in particular, for washing milled products.

The temperature during washing is preferably from 30 to 95°C, more preferably from 50 to 90°C, and still more preferably from 70 to 90°C. The washing time is preferably from 5 minutes to 10 hours, from 5 minutes to 1 hour, and from 10 to 30 minutes. The washing temperature and washing time can be appropriately selected depending on the amount, shape, and the like of the multilayer container or the milled product.

Note that the method for producing a recycled polyester according to the present invention includes a washing step of washing using an alkaline aqueous solution, but may further include a step of washing using a liquid other than the alkaline aqueous solution. Preferably, washing using water is further implemented. After washing, heating is performed as necessary, and drying is preferable. Implementing the drying step can reduce the amount of moisture in the recycled polyester obtained by the present method, and hence it is possible to provide high quality recycled polyester with high thermal stability and the like. The drying step can be performed by air or hot air from a dryer.

Recovery Step

The recovery step is a step in which the multilayer container is milled and recycled polyester is recovered.

In particular, after the multilayer container has been milled, it is preferable to remove the whole or a part of the polyamide layer and thereby selectively taking out polyester, and it is more preferable to separate the polyester from the polyamide resin constituting the polyamide layer.

The multilayer container can be milled by using a grinder such as a single-screw grinder, a twin-screw grinder, a three-screw grinder, or a cutter mill. The milled product obtained after milling has, for example, a flake shape, a powdered

shape, or a bulk shape. However, a large portion of the multilayer container, such as its body, has a multilayer laminated structure with a thin thickness of several mm or less, and hence a large portion of the milled product is typically flaky. Note that the milled product having a flake shape means a flaky or flat-shaped milled product having a thickness of approximately 2 mm or less.

In the multilayer container, the polyester layer and the polyamide layer are structurally integrated, but these layers are usually not adhered to each other, and in the milling step, the polyester and the polyamide resin are easily separated from each other as separate milled products. In addition, the milled product formed into a flake shape is more likely to be blown up and separated by the airflow of air elutriation described below.

However, the polyester and the polyamide resin are not always completely separable from each other in the milling step, and the milled products are separated into products with a relatively high polyester content and products with a relatively low polyester content and a relatively high polyamide resin content. Note that in the following, for explanatory convenience, the milled product with a relatively high polyester content is simply referred to as the polyester, and the milled product with a relatively high polyamide resin content is simply referred to as the polyamide resin.

As described above, the milled products that have been milled are separated into the polyester and the polyamide resin (Separation step).

As the separation method, it is preferred to use specific gravity separation utilizing a difference in specific gravity of the polyester and the polyamide resin.

That is, the polyamide layer is preferably removed by air elutriation after the multilayer container has been milled.

Specifically, examples of specific gravity separation include air elutriation in which milled products are separated by a wind force. An example of air elutriation includes a method of separating and recovering the milled products in a separation apparatus in which a rotating airflow can be generated, and the milled products are subjected to the airflow generated by the separation apparatus and are separated into milled products that have a large specific gravity or a small specific surface area and descend naturally due to their own weight, and milled products that have a small specific gravity or a large specific surface area and are blown upward by the airflow.

With this method, the milled products of the polyester descend naturally due to their own weight, whereas the milled products of the polyamide resin are blown upward, and whereby the polyester and the polyamide resin can be separated from each other and recovered.

In this type of air elutriation, the same milled product may be repeatedly subjected to a similar operation. For example, the naturally descended milled product may be subjected to air elutriation once again to increase the content percentage of polyester in the recycled polyester.

Note that the separation method is not limited to air elutriation, and examples thereof include a method in which the milled product is immersed in a liquid, such as water, and separated due to a difference in specific gravity against the liquid between the milled products; and a method in which a constant level of vibration is applied to the milled product, thereby separating and sorting the milled products having a different specific gravity from each other; and the like.

Granulation Step

The recovered recycled polyester is preferably granulated and formed into pellets in order to facilitate handling during molding processing, and the like.

The granulation may be implemented before or after the crystallization/solid-phase polymerization step described later, but is preferably implemented before the crystallization/solid-phase polymerization step. When the granulation is implemented before the crystallization/solid-phase polymerization step, handleability in the crystallization/solid-phase polymerization step is also improved.

In the granulation step, the milled product is preferably plasticized and granulated by melt blending. Examples of the granulation apparatus for plasticization and granulation include a single-screw extruder, a twin-screw extruder, and a multi-screw extruder, but any known granulation apparatus can be used. The shape of the pellet is preferably a cylindrical shape, a spherical shape, or an elliptical spherical shape.

The granulation is preferably performed by, for example, extruding the plasticized recycled polyester into a strand, and cooling it in a water tank and at the same time cutting it into pellets using a pelletizer. Pellets taken out from the water tank are usually dried to remove moisture adhered to the surface.

Crystallization/Solid-phase Polymerization Step

After the above-described step of recovering polyester, one or more steps selected from a crystallization step and a solid-phase polymerization step are preferably implemented, and it is more preferable to implement both the crystallization step and the solid-phase polymerization step. The crystallization/solid-phase polymerization step is preferably performed for the pelletized polyester described above, but may be performed for polyester that is not pelletized (e.g., a milled product).

Note that when both crystallization and solid-phase polymerization are performed, solid-phase polymerization is preferably implemented after crystallization of the polyester.

Crystallization of the polyester involves keeping the polyester under constant heating. Crystallization is preferably performed by heating the polyester at, for example, from 100 to 230°C. When the polyester is crystallized, there can be prevented polyester-to-polyester fusion and adhesion of polyester to the inner surface of the apparatus during solid-phase polymerization or molding processing.

The solid-phase polymerization is preferably performed by maintaining a temperature higher than or equal to (the melting point of polyester -80°C) and below the melting point of the polyester for a certain period of time. When the temperature is adjusted to be below the melting point, the polyester is prevented from melting, and for example, the working efficiency is prevented from decreasing due to the polyester adhering to the surface of the apparatus. Moreover, when the temperature is adjusted to be higher than or equal to (the melting point of polyester -80°C), polymerization proceeds at a sufficient polymerization rate, and desired physical properties are easily obtained. As used herein, the wording "(the melting point of polyester -80°C)" means "a temperature lower than the melting point of the polyester by 80°C".

The solid-phase polymerization may be performed under vacuum or under a stream of an inert gas such as nitrogen or argon. In a case where the solid-phase polymerization is performed under vacuum, the degree of vacuum is preferably 1.0 torr or less, more preferably 0.5 torr or less, still more preferably 0.1 torr or less. In addition, in both cases of under vacuum or under a stream of an inert gas such as nitrogen or argon, the concentration of oxygen remaining in the system is preferably reduced as much as possible, and the concentration of oxygen is preferably 300 ppm or less, and more preferably 30 ppm or less. When the oxygen concentration is 30 ppm or less, appearance defects such as yellowing are less likely to occur.

In a case where the solid-phase polymerization is performed under vacuum, uniform heat transfer is preferably maintained while stirring or mixing of the polyester is constantly repeated. In a case where the solid-phase polymerization is carried out in the presence of an inert gas, it is preferable to keep a condition where the surface of polyester is always in contact with a dry gas under a dry gas flow.

Examples of the solid-phase polymerization apparatus for carrying out the crystallization/solid-phase polymerization step include a tumbler-type batch apparatus equipped with a heating jacket, a dry silo-type apparatus provided with inert gas stream equipment, a crystallization apparatus provided with an impeller and a discharge screw therein, and a reactor. Note that the crystallization and the solid-phase polymerization are preferably implemented continuously or simultaneously in the same apparatus.

The heating time for the solid-phase polymerization is determined in a timely manner based on the apparatus and other conditions, but enough time for the polyester to acquire sufficient physical properties is needed to be ensured.

In the solid-phase polymerization, the polyester is kept at a high temperature for a long period of time, and therefore impurities present in the polyester may deteriorate the quality such as color tone. A large portion of the polyamide resin is preferably removed in the removal step described above, and in this case, the deterioration of quality that may occur during solid-phase polymerization is minimized.

When the method for producing a recycled polyester includes a step of removing the polyamide resin, the content of the polyamide resin in the resultant recycled polyester is preferably less than 1 mass%, more preferably less than 0.8 mass%, and even more preferably less than 0.6 mass%. As such, the content of the polyamide resin is reduced and thereby improving the quality of the recycled polyester.

In the method for producing a recycled polyester of the present invention, another step other than the steps described above may be implemented.

The recycled polyester obtained by the present production method can be used in various applications such as resin molded articles and fibers.

## Examples

Hereinafter, the present invention will be described more specifically with reference to Examples and Comparative Examples, but the present invention is not limited to these Examples.

## Raw Materials

A polyester resin, a yellowing inhibitor, and a color tone adjusting agent used in Examples and Comparative Examples are as follows. As the polyamide resin, the resin produced in the following Production Example 1 was used.

### Polyester Resin

PET 1101: Polyclear Refresh PET 1101, polyethylene terephthalate (made by Indorama Corporation)

Phenolic Antioxidant (A)

Irganox 1010: Pentaerythritol Tetrakis[3-(3',5'-di-t-butyl-4'-hydroxyphenyl)propionate] (Trade name: Irganox 1010, made by BASF SE)

Phosphorus-based Antioxidants (Phosphorus-based antioxidants (B) and other phosphorus-based antioxidants other than (B))

Doverphos S9228: Bis(2,4-di-cumylphenyl)pentaerythritol diphosphite (Trade name: Doverphos S9228, made by Dover Chemical Corporation, the phosphorus-based antioxidant (B))
PEP-36: Bis(2,6-di-ter-butyl-4-methylphenyl) pentaerythritol-diphosphite (Trade name: ADK STAB PEP-36, made by ADEKA CORPORATION, the phosphorus-based antioxidant (B))
PEP-8: O,O'-Dioctadecylpentaerythritol bis(phosphite) (Phosphorus-based antioxidant having a pentaerythritol skeleton but not having an aromatic ring, trade name: ADK STAB PEP-8, made by ADEKA CORPORATION, a phosphorus-based antioxidant other than (B))
Irgaphos 168: Tris(2,4-di-tert-butylphenyl)phosphite (Phosphorus-based antioxidant having an aromatic ring but not having a pentaerythritol skeleton, trade name: Irgaphos 168, made by BASF SE, a phosphorus-based antioxidant other than (B))

Aldehyde Catcher (C)

Anthranilamide: 2-aminobenzamide (made by Tokyo Chemical Industry Co., Ltd.)

Polyamide Resin

Production Example 1 (Production of polyamide resin (Y1))

Into a reaction container having an internal volume of 50 liters and equipped with a stirrer, a partial condenser, a total condenser, a thermometer, a dropping funnel, a nitrogen inlet tube, and a strand die were put 15,000 g (102.6 mol) of precisely weighed adipic acid, 13.06 g (123.3 mmol, 151 ppm as a phosphorus atom concentration in the polyamide) of sodium hypophosphite monohydrate ($NaH_2PO_2 \cdot H_2O$), and 6.849 g (83.49 mmol, 0.68 as a ratio of the number of moles relative to the sodium hypophosphite monohydrate) of sodium acetate, and then sufficient nitrogen purging was carried out, after which the contents of the system were heated to 170°C while stirring it under a small nitrogen stream. Thereto was added dropwise 13,896 g (102.0 mol, charged molar ratio: 0.994) of meta-xylylenediamine while stirring it, and the temperature in the system was continuously raised while the generated condensed water was removed from the system to the outside. After completion of the dropwise addition of meta-xylylenediamine, the internal temperature was adjusted to 260°C, and the reaction was continued for 40 minutes. The system was then pressurized with nitrogen, and the polymer was taken out from the strand die and formed into pellets to give about 24 kg of polyamide.

Next, the polyamide was put into a jacketed tumble dryer provided with a nitrogen gas inlet tube, a vacuum line, a vacuum pump, and a thermocouple for measuring the internal temperature, and the inside of the tumble dryer was sufficiently purged with nitrogen gas having a purity of 99 vol% or more while the tumble dryer was rotated at a constant speed, after which the tumbler dryer was heated under the same nitrogen gas stream, and the pellet temperature was increased to 150°C over about 150 minutes. When the pellet temperature reached 150°C, the pressure inside the system was reduced to 1 torr or less. Heating was further continued, and the pellet temperature was raised to 200°C over about 70 minutes, and then held at 200°C for 30 to 45 minutes. Next, nitrogen gas having a purity of 99 vol% or more was introduced into the system, and the system was cooled while the tumble dryer was rotated, whereby a polyamide resin (Y1) was obtained. The concentration of the amino end group was measured and found to be 14.4 μmol/g.

Example 1 [Production of multilayer container and recycled polyester]

1. Production of Polyester Resin Mixture

A polyester resin mixture was obtained by previously dry blending 99.94 parts by mass of a polyester resin (Polyclear Refresh PET 1101), 0.0150 parts by mass of Irganox 1010 (150 ppm in the polyester layer) as the phenolic antioxidant (A), and 0.0450 parts by mass of Doverphos S9228 (450 ppm in the polyester layer) as the phosphorus-based antioxidant (B).

2. Production of Multilayer Container

Preform molding

An injection molding machine (Model DU130CI, made by Sumitomo Heavy Industries, Ltd.) having two injection cylinders and a two-cavity mold (made by Kortec) were used to inject a polyamide resin (Y1) through one injection cylinder and the polyester resin mixture through the other injection cylinder, and a three-layer preform (25 g equivalent setting per preform) including a polyester layer/a polyamide layer/a polyester layer was produced by injection molding such that the mass of the polyamide layer relative to the entire preform was as shown in Table 1, under the following conditions. The shape of the preform had a total length of 95 mm, an outer diameter of 22 mm, and a wall thickness of 4.0 mm. The molding conditions for the three-layer preform were as described below.

Skin-side injection cylinder temperature: 285°C
Core-side injection cylinder temperature (only three-layers): 265°C
In-mold resin flow path temperature: 285°C
Mold cooling water temperature: 15°C
Cycle time: 40 seconds

Bottle molding

The preform obtained in the "Preform molding" section was subjected to biaxial stretch blow molding by a blow molding apparatus (EFB1000ET, made by Frontier Inc.) to obtain a bottle (a hollow multilayer container). The bottle has a total length of 223 mm, an outer diameter of 65 mm, an internal volume of 500 mL, and the bottom had a petaloid shape. No dimples were provided in the body. The biaxial stretch blow molding conditions are as indicated below.

Preform heating temperature: 103°C
Pressure for stretching rod: 0.7 MPa
Primary blow pressure: 1.1 MPa
Secondary blow pressure: 2.5 MPa
Primary blow delay time: 0.30 seconds
Primary blow time: 0.30 seconds
Secondary blow time: 2.0 seconds
Blow exhaust time: 0.6 seconds
Mold temperature: 30°C

3. Production of Recycled Polyester

Washing, recovering, and granulation step

A grinder having a mesh diameter of 10 mm was used to comminute 10 kg of the hollow multilayer container obtained in the section "2. Production of Multilayer Container" described above. Into a container equipped with a stirrer was put 1% aqueous sodium hydroxide solution such that the amount of the solution was 4 L per 1 kg of the resultant flaky milled product, and the flaky milled product was washed under stirring. The washing temperature was 85°C and the washing time was 15 minutes. After removing the washing water, the milled product was placed in water at 45°C having a mass four times the milled product, followed by stirring for 5 minutes. After extracting moisture, water having a mass eight times the milled product was further added, followed by stirring. After extracting moisture, the milled product after washing was dried at 50°C.

To this flake was added a flake of a single-layer container of polyester obtained by the same procedure, and then they were dry blended, followed by twofold dilution.

The dried milled product was extruded by a twin-screw extruder (TEM26SX made by Toshiba Machine Co., Ltd.) at a heater temperature of 280°C and a discharge speed of 20 kg/hour to form a strand, which was cut and pelletized using a pelletizer while being cooled in a water tank. Note that air elutriation of the polyamide layer was not performed.

Crystallization/solid-phase polymerization step

The pellet obtained in the granulation step was heated at 200°C for 7 hours under vacuum at a reduced pressure of 1 torr or less. The pellets after the heat treatment were taken out and used as recycled polyester.

Example 2 to 11 and Comparative Example 1 to 17 [Production of multilayer container and recycled polyester]

Multilayer containers and recycled polyesters were produced in the same manner as in Example 1, except that the phenolic antioxidant (A) and the phosphorus-based antioxidant used in the polyester resin composition (polyester layer) in Example 1 were changed to the types and amounts shown in Table 1 to 3, and the aldehyde catchers (C) in Example 7 to 11 and Comparative Examples 7 to 10, 14 and 15 were the type and amount shown in Table 1 to 3. Note that, in Table 1 to 3, the contents of the phenolic antioxidant (A), the phosphorus-based antioxidant, and the aldehyde catcher (C) are represented by "ppm" (parts per million, ppm by mass). 1 ppm is 0.0001 mass%.

Evaluation Method

Preparation of Sample for Evaluation

A sample for evaluation of the recycled polyester for the measurement of the following haze, b* value and Δb* value was prepared as follows.

The heat-treated pellets of Examples and Comparative Examples were used to produce a plate having a length of 60 mm, a width of 90 mm, and a wall thickness of 3.0 mm by injection molding using an injection molding machine (Model SE130DU-HP made by Sumitomo Heavy Industries, Ltd.) having an injection cylinder under the molding conditions set forth below.

Injection cylinder temperature: 280°C
Mold cooling water temperature: 15°C
Cycle time: 45 seconds

Haze

The haze of the plate was measured as an average value calculated from four measurements using a haze meter COH7700 (White LED light source, made by Nippon Denshoku Industries Co., Ltd.) in accordance with JIS K 7136:2000. The smaller haze value is preferable, since the recycled polyester is more excellent in transparency.

b* value

The b* value of the sample for evaluation was measured using a haze meter COH400 (made by Nippon Denshoku Industries Co., Ltd.) in accordance with JIS K 7105:1981.

Note that the b*value represents the chromaticity. +b* direction indicates the yellow color direction, and -b* direction indicates the blue color direction. The smaller the absolute value of the b* value is and the smaller the b* value is, yellowing is more suppressed, and more excellent in colorlessness and more excellent in color tone.

Δb* value

The polyester resin (Polyclear Refresh PET 1101) used as the raw material was molded under the molding conditions as indicted in the "Preparation of Sample for Evaluation" section to produce a plate having a length of 60 mm, a width of 90 mm, and a wall thickness of 3.0 mm. Next, the b* value thereof was measured using a haze meter COH400 (made by Nippon Denshoku Industries Co., Ltd.) in accordance with JIS K 7105 : 1981 (b* value was 4.1).

The difference between the b* value of the samples for evaluation of Examples and Comparative Examples obtained in the section <b* value> described above and the b* value (4.1) of the polyester resin used as the raw material was defined as Δb* value. The smaller Δb* value is preferable, since yellowing is further suppressed, the discoloration from the raw material polyester resin is smaller, and colorlessness is more excellent. Note that, in this evaluation, colorlessness is good if the Δb* value is less than 4.5, and colorlessness is poor if the Δb* value is 4.5 or more.

[Table 1]

Table 1

| | | | | Examples | | | | | | | | | | |
|---|---|---|---|---|---|---|---|---|---|---|---|---|---|---|
| | | | | 1 | 2 | 3 | 4 | 5 | 6 | 7 | 8 | 9 | 10 | 11 |
| Polyester resin composition | Phenolic antioxidant (A) | Irganox1010 | ppm | 150 | 240 | 400 | 500 | 240 | 500 | 100 | 240 | 400 | 240 | 240 |
| | Phosphorus-based antioxidant | Doverphos S9228 | ppm | 450 | 720 | 1200 | 1500 | - | - | 300 | 720 | 1200 | - | 720 |
| | | PEP36 | ppm | - | - | - | - | 720 | 1500 | - | - | - | 720 | - |
| | | PEP8 | ppm | - | - | - | - | - | - | - | - | - | - | - |
| | | Irgafos168 | ppm | - | - | - | - | - | - | - | - | - | - | - |
| | Aldehyde catcher (C) | Anthranilamide | ppm | - | - | - | - | - | - | 500 | 500 | 500 | 500 | 50 |
| | Total content of antioxidants | | ppm | 600 | 960 | 1600 | 2000 | 960 | 2000 | 400 | 960 | 1600 | 960 | 960 |
| | Total content of antioxidant and aldehyde catcher | | ppm | 600 | 960 | 1600 | 2000 | 960 | 2000 | 900 | 1460 | 2100 | 1460 | 1010 |
| | Polyester resin (PET 1101) | | mass% | Balance | Balance | Balance | Balance | Balance | Balance | Balance | Balance | Balance | Balance | Balance |
| | Total | | mass% | 100 | 100 | 100 | 100 | 100 | 100 | 100 | 100 | 100 | 100 | 100 |
| Multilayer container | Polyester layer | Polyester resin composition | mass% | 94 | 94 | 94 | 94 | 94 | 94 | 94 | 94 | 94 | 94 | 94 |
| | Polyamide layer | Polyamide resin (Y1) | mass% | 6 | 6 | 6 | 6 | 6 | 6 | 6 | 6 | 6 | 6 | 6 |
| | Total | | mass% | 100 | 100 | 100 | 100 | 100 | 100 | 100 | 100 | 100 | 100 | 100 |
| Evaluation of recycled polyester | Haze | | % | 18.4 | 21.0 | 22.0 | 29.8 | 20.8 | 27.7 | 17.4 | 21.6 | 25.4 | 20.8 | 19.2 |
| | b* | | - | 8.4 | 8.1 | 8.0 | 7.9 | 8.3 | 8.3 | 6.8 | 5.5 | 6.4 | 5.9 | 7.9 |
| | Δb* | | - | 4.3 | 4.0 | 3.9 | 3.8 | 4.2 | 4.2 | 2.7 | 1.4 | 2.3 | 1.8 | 3.8 |

[Table 2]

Table 2

| | | | | Comparative Examples | | | | | | | | |
|---|---|---|---|---|---|---|---|---|---|---|---|---|
| | | | | 1 | 2 | 3 | 4 | 5 | 6 | 7 | 8 | 9 |
| Polyester resin composition | Phenolic antioxidant (A) | Irganox1010 | ppm | - | 500 | 2000 | - | - | - | - | - | 500 |
| | Phosphorus-based antioxidant | Doverphos S9228 | ppm | - | - | - | 1500 | 3010 | - | - | - | - |
| | | PEP36 | ppm | - | - | - | - | - | 1500 | - | - | - |
| | | PEP8 | ppm | - | - | - | - | - | - | - | - | - |
| | | Irgafos168 | ppm | - | - | - | - | - | - | - | - | - |
| | Aldehyde catcher (C) | Anthranilamide | ppm | - | - | - | - | - | - | 500 | 1500 | 500 |
| | Total content of antioxidants | | ppm | 0 | 500 | 2000 | 1500 | 3010 | 1500 | 0 | 0 | 500 |
| | Total content of antioxidant and aldehyde catcher | | ppm | 0 | 500 | 2000 | 1500 | 3010 | 1500 | 500 | 1500 | 1000 |
| | Polyester resin (PET 1101) | | mass% | Balance | Balance | Balance | Balance | Balance | Balance | Balance | Balance | Balance |
| | Total | | mass% | 100 | 100 | 100 | 100 | 100 | 100 | 100 | 100 | 100 |
| Multilayer container | Polyester layer | Polyester resin composition | mass% | 94 | 94 | 94 | 94 | 94 | 94 | 94 | 94 | 94 |
| | Polyamide layer | Polyamide resin (Y1) | mass% | 6 | 6 | 6 | 6 | 6 | 6 | 6 | 6 | 6 |
| | Total | | mass% | 100 | 100 | 100 | 100 | 100 | 100 | 100 | 100 | 100 |
| Evaluation of recycled polyester | Haze | | % | 20.2 | 21.3 | 22.2 | 18.7 | 23.1 | 19.9 | 19.8 | 19.3 | 19.2 |
| | b* | | - | 11.8 | 9.8 | 9.4 | 9.8 | 9.4 | 9.8 | 10.3 | 10.0 | 9.1 |
| | Δb* | | - | 7.7 | 5.7 | 5.3 | 5.6 | 5.3 | 5.7 | 6.2 | 5.9 | 5.0 |

[Table 3]

Table 3

| | | | | Comparative Examples | | | | | | | |
|---|---|---|---|---|---|---|---|---|---|---|---|
| | | | | 10 | 11 | 12 | 13 | 14 | 15 | 16 | 17 |
| Polyester resin composition | Phenolic antioxidant (A) | Irganox1010 | ppm | - | 100 | 600 | 800 | 240 | 500 | 500 | 500 |
| | Phosphorus-based antioxidant | Doverphos S9228 | ppm | 1500 | 300 | 1800 | 2410 | 720 | 1500 | - | - |
| | | PEP36 | ppm | - | - | - | - | - | - | - | - |
| | | PEP8 | ppm | - | - | - | - | - | - | 1500 | - |
| | | Irgafos168 | ppm | - | - | - | - | - | - | - | 1500 |
| | Aldehyde catcher (C) | Anthranilamide | ppm | 500 | - | - | - | 1500 | 500 | - | - |
| | Total content of antioxidants | | ppm | 1500 | 400 | 2400 | 3210 | 960 | 2000 | 2000 | 2000 |
| | Total content of antioxidant and aldehyde catcher | | ppm | 2000 | 400 | 2400 | 3210 | 2460 | 2500 | 2000 | 2000 |
| | Polyester resin (PET 1101) | | mass% | Balance | Balance | Balance | Balance | Balance | Balance | Balance | Balance |
| | Total | | mass% | 100 | 100 | 100 | 100 | 100 | 100 | 100 | 100 |
| Multilayer container | Polyester layer | Polyester resin composition | mass% | 94 | 94 | 94 | 94 | 94 | 94 | 94 | 94 |
| | Polyamide layer | Polyamide resin (Y1) | mass% | 6 | 6 | 6 | 6 | 6 | 6 | 6 | 6 |
| | Total | | mass% | 100 | 100 | 100 | 100 | 100 | 100 | 100 | 100 |
| Evaluation of recycled polyester | Haze | | % | 20.1 | 20.0 | 35.4 | 37.8 | 32.4 | 39.8 | 19.5 | 19.6 |
| | b* | | - | 9.1 | 10.4 | 8.7 | 9.5 | 6.5 | 7.4 | 18.9 | 9.7 |
| | Δb* | | - | 5.0 | 6.3 | 4.6 | 5.4 | 2.4 | 3.3 | 14.8 | 5.6 |

As shown in Table 1, it can be seen that the recycled polyester obtained by recycling the multilayer container of the present invention has a low yellowness, is excellent in colorlessness, and is also excellent in transparency.

## Claims

1. A multilayer container, comprising: a polyester layer containing a polyester resin (X), a phenolic antioxidant (A), and a phosphorus-based antioxidant (B); or a polyester layer containing a polyester resin (X), a phenolic antioxidant (A), a phosphorus-based antioxidant (B), and an aldehyde catcher (C); and a polyamide layer containing a polyamide resin (Y), wherein a total content of the phenolic antioxidant (A), the phosphorus-based antioxidant (B), and the aldehyde catcher (C) in the polyester layer is from 0.05 to 0.22 mass%, and the phosphorus-based antioxidant (B) is a compound having a pentaerythritol skeleton and an aromatic ring.

2. The multilayer container according to claim 1, wherein a total content of the phenolic antioxidant (A) and the phosphorus-based antioxidant (B) in the polyester layer is from 0.05 to 0.22 mass%.

3. The multilayer container according to claim 1 or 2, wherein a mass ratio [(A)/(B)] of a content of the phenolic antioxidant (A) to a content of the phosphorus-based antioxidant (B) is from 2/8 to 5/5.

4. The multilayer container according to any one of claims 1 to 3, wherein the polyester layer contains an aldehyde catcher (C).

5. The multilayer container according to any one of claims 1 to 4, wherein the aldehyde catcher (C) is anthranilamide.

6. The multilayer container according to any one of claims 1 to 5, wherein a content of the aldehyde catcher (C) in the polyester layer is from 0.005 to 0.120 mass%.

7. The multilayer container according to any one of claims 1 to 6, wherein the polyamide resin (Y) contains a structural unit derived from a diamine and a structural unit derived from a dicarboxylic acid, 80 mol% or more of the structural unit derived from a diamine being a structural unit derived from xylylenediamine, and 80 mol% or more of the structural unit derived from a dicarboxylic acid being a structural unit derived from adipic acid.

8. The multilayer container according to any one of claims 1 to 7, wherein a content of the polyamide resin (Y) is from 0.05 to 10.0 mass% relative to a total amount of all polyamide layers and all polyester layers.

9. The multilayer container according to any one of claims 1 to 8, wherein a content of the polyamide layer is from 0.05 to 10.0 mass% relative to a total amount of all polyamide layers and all polyester layers.

10. The multilayer container according to any one of claims 1 to 9, wherein the polyester resin (X) contains a structural unit derived from a dicarboxylic acid and a structural unit derived from a diol, 80 mol% or more of the structural unit derived from a dicarboxylic acid being a structural unit derived from terephthalic acid, and 80 mol% or more of the structural unit derived from a diol being a structural unit derived from ethylene glycol.

11. The multilayer container according to any one of claims 1 to 10, wherein the multilayered container is a hollow container.

12. The multilayer container according to any one of claims 1 to 11, wherein the multilayer container has a three- to five-layer structure, and the outermost layer and the innermost layer are polyester layers.

13. A multilayer container, comprising: a polyester layer containing a polyester resin (X), a phenolic antioxidant (A), a phosphorus-based antioxidant (B), and an aldehyde catcher (C); and a polyamide layer containing a polyamide resin (Y),

    wherein the phosphorus-based antioxidant (B) is a compound having a pentaerythritol skeleton and an aromatic ring, and the aldehyde catcher (C) is anthranilamide,
    a total content of the phenolic antioxidant (A), the phosphorus-based antioxidant (B), and the aldehyde catcher (C) in the polyester layer is from 0.05 to 0.22 mass%,
    a content of the aldehyde catcher (C) in the polyester layer is from 0.005 to 0.120 mass%, and a mass ratio

[(A)/(B)] of a content of the phenolic antioxidant (A) to a content of the phosphorus-based antioxidant (B) is from 2/8 to 5/5.

**14.** A method for producing a recycled polyester, the method comprising recovering polyester from the multilayer container according to any one of claims 1 to 13.

**15.** The method for producing a recycled polyester according to claim 14, wherein the polyester is subjected to one or more steps selected from crystallizing and solid-phase polymerizing after the recovering.

**16.** The method for producing a recycled polyester according to claim 14 or 15, the method comprising removing the whole or a part of the polyamide layer from the multilayer container to recover the polyester.

**17.** The method for producing a recycled polyester according to claim 16, wherein the removing of the polyamide layer is conducted by, after milling of the multilayer container, air elutriation.

**18.** The method for producing a recycled polyester according to any one of claims 15 to 17, wherein the method comprises washing the multilayer container or a milled product thereof with an alkaline aqueous solution to recover the polyester.

## INTERNATIONAL SEARCH REPORT

International application No.
**PCT/JP2024/007032**

### A. CLASSIFICATION OF SUBJECT MATTER

***B65D 1/00***(2006.01)i; ***B29B 17/04***(2006.01)i; ***B32B 1/00***(2024.01)i; ***B32B 27/18***(2006.01)i; ***B32B 27/34***(2006.01)i; ***B32B 27/36***(2006.01)i; ***B65D 65/40***(2006.01)i; ***C07C 237/30***(2006.01)i; ***C07F 9/6574***(2006.01)i; ***C08J 11/04***(2006.01)i
FI: B65D1/00 111; B65D65/40 D; B32B1/00 Z; B32B27/18 Z; B32B27/34; B32B27/36; C07F9/6574; B29B17/04; C08J11/04; C07C237/30; B65D1/00 ZAB

According to International Patent Classification (IPC) or to both national classification and IPC

### B. FIELDS SEARCHED

Minimum documentation searched (classification system followed by classification symbols)

B65D1/00; B29B17/04; B32B1/00; B32B27/18; B32B27/34; B32B27/36; B65D65/40; C07C237/30; C07F9/6574; C08J11/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2022-86561 A (MITSUBISHI GAS CHEMICAL COMPANY, INC.) 09 June 2022 (2022-06-09) paragraphs [0009]-[0012], [0022]-[0026], [0042]-[0045], [0069], [0076], [0082] | 1-18 |
| Y | JP 2013-39964 A (MITSUBISHI GAS CHEMICAL COMPANY, INC.) 28 February 2013 (2013-02-28) paragraphs [0055]-[0058] | 1-18 |
| Y | JP 2015-142986 A (MITSUBISHI GAS CHEMICAL COMPANY, INC.) 06 August 2015 (2015-08-06) paragraphs [0119]-[0122] | 1-18 |
| Y | JP 2012-520364 A (BASF SE) 06 September 2012 (2012-09-06) paragraphs [0038]-[0040], [0100]-[0101] | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 April 2024** | **23 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No. **PCT/JP2024/007032**

**C. DOCUMENTS CONSIDERED TO BE RELEVANT**

| Category* | Citation of document, with indication, where appropriate, of the relevant passages | Relevant to claim No. |
|---|---|---|
| Y | JP 2010-215760 A (DAIWA CAN CO., LTD.) 30 September 2010 (2010-09-30) paragraph [0031] | 3-18 |
| Y | JP 2017-178439 A (MITSUBISHI GAS CHEMICAL COMPANY, INC.) 05 October 2017 (2017-10-05) paragraph [0065] | 6-18 |
| Y | JP 2021-127173 A (MITSUBISHI GAS CHEMICAL COMPANY, INC.) 02 September 2021 (2021-09-02) paragraphs [0108]-[0112] | 14-18 |
| Y | JP 2018-43773 A (MITSUBISHI GAS CHEMICAL COMPANY, INC.) 22 March 2018 (2018-03-22) paragraph [0071] | 18 |

Form PCT/ISA/210 (second sheet) (July 2022)

**INTERNATIONAL SEARCH REPORT**
**Information on patent family members**

International application No.
**PCT/JP2024/007032**

| Patent document cited in search report | Publication date (day/month/year) | Patent family member(s) | Publication date (day/month/year) |
|---|---|---|---|
| JP 2022-86561 A | 09 June 2022 | (Family: none) | |
| JP 2013-39964 A | 28 February 2013 | (Family: none) | |
| JP 2015-142986 A | 06 August 2015 | (Family: none) | |
| JP 2012-520364 A | 06 September 2012 | US 2010/0233405 A1<br>paragraphs [0002], [0054]-[0057], [0119]<br>WO 2010/103023 A1<br>CN 102348762 A<br>KR 10-2012-0014889 A | |
| JP 2010-215760 A | 30 September 2010 | (Family: none) | |
| JP 2017-178439 A | 05 October 2017 | (Family: none) | |
| JP 2021-127173 A | 02 September 2021 | US 2023/0102641 A1<br>paragraphs [0290]-[0308]<br>WO 2021/161844 A1<br>EP 4105020 A1<br>CN 114981081 A<br>KR 10-2022-0139889 A | |
| JP 2018-43773 A | 22 March 2018 | US 2018/0072844 A1<br>paragraph [0181]<br>EP 3296097 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018043773 A